(19) Europäisches Patentamt
European Patent Office
Office européen des brevets

(11) EP 4 083 088 A1

(12) EUROPEAN PATENT APPLICATION
published in accordance with Art. 153(4) EPC

(43) Date of publication:
**02.11.2022 Bulletin 2022/44**

(21) Application number: **20907516.7**

(22) Date of filing: **25.12.2020**

(51) International Patent Classification (IPC):
***C08F 220/10*** *(2006.01)* ***B33Y 10/00*** *(2015.01)*
***B33Y 80/00*** *(2015.01)* ***B29C 64/106*** *(2017.01)*
***B29C 64/259*** *(2017.01)* ***B29C 64/264*** *(2017.01)*
***A61K 6/30*** *(2020.01)* ***A61K 6/889*** *(2020.01)*
***B33Y 70/00*** *(2020.01)*

(52) Cooperative Patent Classification (CPC):
**A61K 6/30; A61K 6/889; B29C 64/106; B29C 64/259; B29C 64/264; B33Y 10/00; B33Y 70/00; B33Y 80/00; C08F 220/10**

(86) International application number:
**PCT/JP2020/049041**

(87) International publication number:
**WO 2021/132699 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2019 JP 2019234880**

(71) Applicant: **Kuraray Noritake Dental Inc.**
**Kurashiki-shi, Okayama 710-0801 (JP)**

(72) Inventors:
- **ITO, Misaki**
  **Tainai-shi, Niigata 959-2691 (JP)**
- **SUZUKI, Kenji**
  **Tainai-shi, Niigata 959-2691 (JP)**

(74) Representative: **D Young & Co LLP**
**120 Holborn**
**London EC1N 2DY (GB)**

(54) **ENERGY RAY-CURABLE COATING MATERIAL FOR THREE-DIMENSIONAL MOLDING, ENERGY RAY-CURABLE MATERIAL KIT FOR THREE-DIMENSIONAL MOLDING CONTAINING SAID SAME, THREE-DIMENSIONAL MOLDED PRODUCT OBTAINED USING SAME, AND METHOD FOR PRODUCING SAME**

(57) The present invention provides an energy ray-curable coating material for three-dimensional shaped articles that provides excellent toughness in the cured product, and an energy ray-curable material kit for three-dimensional shaping including the coating material. The present invention relates to an energy ray-curable coating material (A) for three-dimensional shaped articles, comprising a polymerizable compound and a polymerization initiator (c), the polymerizable compound comprising a monofunctional polymerizable compound (a), and/or a polyfunctional polymerizable compound (b) having two or more polymerizable groups per molecule, the polyfunctional polymerizable compound (b) having a Mw/n of 120 or more, where Mw is a molecular weight of the polyfunctional polymerizable compound (b), and n is the number of polymerizable groups per molecule.

EP 4 083 088 A1

## Description

TECHNICAL FIELD

The present invention relates to an energy ray-curable coating material for three-dimensional shaped articles, and an energy ray-curable material kit for three-dimensional shaping configured from the coating material and an energy ray-curable composition for three-dimensional shaping. The invention also relates to a three-dimensional shaped article coated with the coating material, and a method of production of such a three-dimensional shaped article.

BACKGROUND ART

Stereolithography has attracted interest as a technique of fabricating a shaped article by laminating a layer of a photocurable resin composition cured by laser scanning or projection of a beam by a projector method based on three-dimensional CAD data. The energy ray-curable three-dimensional shaping technique (hereinafter, "energy ray-curable three-dimensional shaping" is also referred to as "three-dimensional shaping") enables easy and quick fabrication of a prototype without using a die or a mold, and can save time and cost of product development from designing to production. The rapid spread of three-dimensional CAD has allowed the three-dimensional shaping technique to be used in a wide range of fields in industry, including automobile parts, electrical devices, and medical equipment.

The improvement and increasing applications of the three-dimensional shaping technique have led to attempts to apply this technique to final products, beyond prototypes, raising the levels of performance required for energy ray-curable compositions for three-dimensional shaping. Milling is incapable in elaborate working of a flexible material, or working of a final product of a shape to which a drill is not easily accessible, such as in a hollow shape. There accordingly is a need for an energy ray-curable resin that can be formed into a shaped article with excellent precision upon cure while being flexible.

Aside from flexibility, the property to resist breakage (high toughness) is a common requirement for dental materials that may contact soft tissues such as a mucosa and the tongue, for example, such as an orthodontic mouthpiece used for orthodontic treatment (e.g., an aligner, a retainer), a mouthpiece for the treatment of sleep disorders or temporomandibular joint disorders, a mouthpiece for protecting teeth or temporomandibular joint (e.g., an occlusal splint, a mouthguard), and a denture base material. While a loss of flexibility creates discomfort, susceptibility to breakage leads to problems such as frequent replacements, or involves a risk of aspiration or intraoral injuries.

Surface smoothness represents another requirement for such materials, particularly materials that potentially contact soft tissues. A loss of surface smoothness is problematic because it can be perceived as the presence of a foreign object by patient, or may present poor aesthetics because of lack of glossiness. Materials with no surface smoothness also suffer from poor durability because portions with irregularities are susceptible to damage. Other problems include reduced efficiency of orthodontic treatment as a result of reduced adherence, and health issues such as dental caries and bad breath due to the food residue or adhesion of bacteria.

However, shaped articles formed by three-dimensional shaping inevitably involve unevenness due to their layered structure. In this regard, methods are proposed that are intended to improve such unevenness (Patent Literatures 1 and 2).

Patent Literature 1 discloses a method for post-processing of a shaped article formed by stereolithography, whereby the surface of a stereolithographically formed article to be used as a mold is coated with a photocurable or thermosetting resin composition, and the curable resin composition is cured to smooth the unevenness in the surface of the shaped article. In Patent Literature 2, a method for producing a product such as a shell for hearing aids is disclosed that comprises coating a photocurable or thermosetting resin composition on a stereolithographically formed article to be used as a product such as a shell for hearing aids, and curing the coated curable resin composition.

CITATION LIST

Patent Literature

Patent Literature 1: JP 2000-318048 A
Patent Literature 2: JP 2002-305796 A

SUMMARY OF INVENTION

Technical Problem

However, Patent Literature 1 does not disclose details concerning preferred forms of polymerizable compounds used for the curable resin composition. After studies, the present inventors also found that, when a curable composition formed of a low-molecular bifunctional polymerizable compound such as that used in Examples is used as a coating material of a shaped article having flexibility, the coating easily becomes detached or broken in response to deformation of the article. Patent Literature 2 is intended for hard shaped articles, such as a shell of hearing aids. The narrow deformable range of a hard shaped article allows a common coating material to impart smoothness without impairing the article's intended functions. However, because a flexible shaped article has a wider deformable range, it is not easy to coat a flexible shaped article without impairing the inherent properties of the article. For example, when the coating material used is hard and brittle such as a common hard coating agent, the coating tends to break by failing to follow deformation when a flexible shaped article undergoes large deformation. In order for a coating to smooth the surface of a flexible and highly deformable shaped article without impairing the properties of the article, the coating must have high rigidity while being able to stretch after yielding to resist fracture. That is, the coating must excel in toughness.

It is accordingly an object of the present invention to provide an energy ray-curable coating material for three-dimensional shaped articles that provides excellent toughness in the cured product. Another object of the present invention is to provide a kit including the coating material and an energy ray-curable composition for three-dimensional shaping. Yet another object of the present invention is to provide an orthodontic mouthpiece, a mouthpiece for treating sleep disorders, a mouthpiece for treating temporomandibular joint disorders, a mouthpiece for protection of teeth or temporomandibular joint, and a denture base material formed of a three-dimensional shaped article fabricated by using the kit.

Solution to Problem

Specifically, the present invention includes the following.

[1] An energy ray-curable coating material (A) for three-dimensional shaped articles, comprising a polymerizable compound and a polymerization initiator (c),

the polymerizable compound comprising a monofunctional polymerizable compound (a), and/or a polyfunctional polymerizable compound (b) having two or more polymerizable groups per molecule,
the polyfunctional polymerizable compound (b) having a Mw/n of 120 or more, where Mw is a molecular weight of the polyfunctional polymerizable compound (b), and n is the number of polymerizable groups per molecule.

[2] The energy ray-curable coating material (A) for three-dimensional shaped articles according to [1], wherein the polymerization initiator (c) is a photopolymerization initiator (c1).
[3] The energy ray-curable coating material (A) for three-dimensional shaped articles according to [1] or [2], wherein the polymerizable compound comprises the polyfunctional polymerizable compound (b), and the polyfunctional polymerizable compound (b) comprises a polyfunctional polymerizable compound having a Mw/n of 500 or more.
[4] The energy ray-curable coating material (A) for three-dimensional shaped articles according to any one of [1] to [3], wherein the polymerizable compound comprises the monofunctional polymerizable compound (a), and the monofunctional polymerizable compound (a) is a radical polymerizable compound.
[5] The energy ray-curable coating material (A) for three-dimensional shaped articles according to any one of [1] to [4], wherein the monofunctional polymerizable compound (a) comprises a monofunctional polymerizable compound (a1) having a polymer glass transition temperature of 60°C or more.
[6] The energy ray-curable coating material (A) for three-dimensional shaped articles according to [5], wherein the monofunctional polymerizable compound (a1) is a (meth)acrylic compound having a side chain having at most two carbon atoms.
[7] The energy ray-curable coating material (A) for three-dimensional shaped articles according to [5], wherein the monofunctional polymerizable compound (a1) is an aromatic (meth)acrylate compound.
[8] The energy ray-curable coating material (A) for three-dimensional shaped articles according to any one of [1] to [7], wherein the monofunctional polymerizable compound (a) comprises a monofunctional polymerizable compound (a2) having a polymer glass transition temperature of less than 60°C.
[9] The energy ray-curable coating material (A) for three-dimensional shaped articles according to any one of [1] to [7], wherein the polyfunctional polymerizable compound (b) comprises a polyfunctional polymerizable compound (b1) having a polymer glass transition temperature of 60°C or more.

[10] The energy ray-curable coating material (A) for three-dimensional shaped articles according to [9], wherein the polyfunctional polymerizable compound (b1) comprises a polyfunctional polymerizable compound (b1-1) having no polymer backbone.
[11] The energy ray-curable coating material (A) for three-dimensional shaped articles according to [9], wherein the polyfunctional polymerizable compound (b1) comprises a polyfunctional polymerizable compound (b1-2) having a polymer backbone.
[12] The energy ray-curable coating material (A) for three-dimensional shaped articles according to any one of [1] to [11], wherein the monofunctional polymerizable compound (a) and/or the polyfunctional polymerizable compound (b) have at least one selected from an alicyclic hydrocarbon group, an aromatic hydrocarbon group, a chain alkyl group having 8 or more carbon atoms, a fluoro group, and a silyl group.
[13] The energy ray-curable coating material (A) for three-dimensional shaped articles according to any one of [1] to [12], wherein the monofunctional polymerizable compound (a) and/or the polyfunctional polymerizable compound (b) have a zwitterionic group and/or a quaternary ammonium salt group.
[14] The energy ray-curable coating material (A) for three-dimensional shaped articles according to any one of [1] to [13], which further comprises a solvent (d).
[15] An energy ray-curable material kit for three-dimensional shaping, comprising an energy ray-curable coating material (A) for three-dimensional shaped articles of any one of [1] to [14], and an energy ray-curable composition (B) for three-dimensional shaping.
[16] The energy ray-curable material kit for three-dimensional shaping according to [15], wherein the energy ray-curable composition (B) for three-dimensional shaping comprises a polymerizable compound and a polymerization initiator (c),

the polymerizable compound comprising a monofunctional polymerizable compound (a), and/or a polyfunctional polymerizable compound (b) having two or more polymerizable groups per molecule,
the polyfunctional polymerizable compound (b) having a Mw/n of 120 or more, where Mw is a molecular weight of the polyfunctional polymerizable compound (b), and n is the number of polymerizable groups per molecule.

[17] The energy ray-curable material kit for three-dimensional shaping according to [15] or [16], wherein the difference between a transparency $\Delta L_A$ of a cured product of the energy ray-curable coating material (A) for three-dimensional shaped articles and a transparency $\Delta L_B$ of a cured product of the energy ray-curable composition (B) for three-dimensional shaping is 20 or less.
[18] The energy ray-curable material kit for three-dimensional shaping according to any one of [15] to [17], wherein the difference between a tensile elastic modulus of a cured product of the energy ray-curable coating material (A) for three-dimensional shaped articles and a tensile elastic modulus of a cured product of the energy ray-curable composition (B) for three-dimensional shaping is 200 MPa or less.
[19] A three-dimensional shaped article comprising a coating layer,

the three-dimensional shaped article being a cured product of an energy ray-curable composition (B) for three-dimensional shaping,
the coating layer being a cured product of an energy ray-curable coating material (A) for three-dimensional shaped articles of any one of [1] to [14].

[20] The three-dimensional shaped article according to [19], wherein the coating layer formed of the energy ray-curable coating material (A) for three-dimensional shaped articles is 250 $\mu$m or less.
[21] The three-dimensional shaped article according to [19] or [20], wherein a covalent bond is formed between the polymerizable compound in the energy ray-curable coating material (A) for three-dimensional shaped articles constituting the coating layer, and a polymerizable compound in the energy ray-curable composition (B) for three-dimensional shaping constituting the three-dimensional shaped article.
[22] A method for producing a coated three-dimensional shaped article, comprising the steps of:

curing the energy ray-curable composition (B) for three-dimensional shaping included in the energy ray-curable material kit for three-dimensional shaping of any one of [15] to [18] to form a three-dimensional shaped article; and
coating the three-dimensional shaped article with the energy ray-curable coating material (A) for three-dimensional shaped articles included in the energy ray-curable material kit for three-dimensional shaping.

[23] The method for producing a coated three-dimensional shaped article according to [22], which further comprises subjecting the three-dimensional shaped article coated with the energy ray-curable coating material (A) for three-dimensional shaped articles to photoirradiation and/or heat treatment to integrate the energy ray-curable coating

material (A) for three-dimensional shaped articles and the three-dimensional shaped article of the energy ray-curable composition (B) for three-dimensional shaping.

[24] The method for producing a coated three-dimensional shaped article according to [22] or [23], wherein the energy ray-curable composition (B) for three-dimensional shaping is cured by stereolithography.

[25] The method for producing a coated three-dimensional shaped article according to any one of [22] to [24], wherein the three-dimensional shaped article is a dental material.

[26] The method for producing a coated three-dimensional shaped article according to any one of [22] to [25], wherein the three-dimensional shaped article is an orthodontic mouthpiece, a mouthpiece for treating sleep disorders, a mouthpiece for treating temporomandibular joint disorders, or a mouthpiece for protecting teeth or temporomandibular joint.

[27] The method for producing a coated three-dimensional shaped article according to [25], wherein the dental material is a denture base material.

Advantageous Effects of Invention

According to the present invention, an energy ray-curable coating material for three-dimensional shaped articles can be provided that provide excellent toughness in the cured product. This makes the invention suitable for use in smoothing the surface of an energy ray-curable three-dimensional shaped article having flexibility. In other words, the present invention can impart smoothness to the surface of a flexible three-dimensional shaped article made of resin, without impairing the properties of the shaped article. The present invention can also provide a kit including the coating material and an energy ray-curable composition for three-dimensional shaping. The present invention can also provide an orthodontic mouthpiece, a mouthpiece for treating sleep disorders, a mouthpiece for treating temporomandibular joint disorders, a mouthpiece for protection of teeth or temporomandibular joint, and a denture base material formed of a three-dimensional shaped article fabricated by using the kit.

DESCRIPTION OF EMBODIMENTS

Energy Ray-Curable Coating Material (A) for Three-Dimensional Shaped Articles

An energy ray-curable coating material (A) for three-dimensional shaped articles of the present invention (hereinafter, also referred to simply as "coating material (A)") comprises a monofunctional polymerizable compound (a) and/or a polyfunctional polymerizable compound (b) having a Mw/n of 120 or more, and a polymerization initiator (c), the polyfunctional polymerizable compound (b) having a Mw/n of 120 or more, where Mw is a molecular weight of the polyfunctional polymerizable compound (b), and n is the number of polymerizable groups per molecule.

Monofunctional Polymerizable Compound (a)

In an energy ray-curable coating material (A) for three-dimensional shaped articles of the present invention, the monofunctional polymerizable compound (a) is used to impart toughness to a cured product of the coating material (A). Because the monofunctional polymerizable compound (a) does not form crosslinks upon cure, the distances between crosslinking points are not too short in the cured product, and deformation more easily takes place when the monofunctional polymerizable compound (a) is present in the coating material (A). Depending on the type of side chain, the monofunctional polymerizable compound (a) can also impart strength and flexibility to a cured product of the coating material (A).

Preferred for use as the monofunctional polymerizable compound (a) are radical polymerizable compounds. Specific examples of the radical polymerizable compounds include (meth)acrylate compounds; (meth)acrylamide compounds; (meth)acrylic acid, $\alpha$-cyanoacrylic acid, $\alpha$-halogenated acrylic acid, crotonic acid, cinnamic acid, sorbic acid, maleic acid, itaconic acid and esters of these; vinyl esters; vinyl ethers; mono-N-vinyl derivatives; and styrene derivatives. In view of curability, preferred are (meth)acrylate compounds and (meth)acrylamide compounds.

The monofunctional polymerizable compound (a) includes a monofunctional polymerizable compound (a1) having a polymer glass transition temperature (Tg) of 60°C or more (hereinafter, also referred to simply as "monofunctional polymerizable compound (a1)"), and a monofunctional polymerizable compound (a2) having a polymer glass transition temperature of less than 60°C (hereinafter, also referred to simply as "monofunctional polymerizable compound (a2)"). In view of toughness of a polymer of the monofunctional polymerizable compound (a) constituting a cured product of coating material (A), it is preferable to use the monofunctional polymerizable compound (a1) and the monofunctional polymerizable compound (a2) in combination. In this specification, the "polymer glass transition temperature" of a given compound means a glass transition temperature of a homopolymer of the compound, and the glass transition temperature can be measured with a viscoelasticity meter (rheometer) using a conventionally known method. For example, glass

transition temperature (Tg) can be measured by a dynamic viscoelasticity measurement of (meth)acrylic compound (A) performed with a rotary rheometer (AR 2000, manufactured by TA Instruments Japan Inc.) at 10 Hz frequency under a 10 N load with 0.1% displacement and 20 μNm torque, and the temperature at which tanΔ takes a peak can be determined as the glass transition temperature (Tg). In this specification, "polymer glass transition temperature" is also referred to more conveniently as "glass transition temperature", "Tg", or "polymer Tg".

The monofunctional polymerizable compound (a1) is not particularly limited, as long as it has a glass transition temperature of 60°C or more. Examples of the monofunctional polymerizable compound (a1) include:

(meth)acrylate compounds such as aliphatic (meth)acrylate compounds (e.g., methyl methacrylate, ethyl methacrylate, t-butyl (meth)acrylate, 4-t-butylcyclohexyl (meth)acrylate, cyclohexyl (meth)acrylate, isobornyl (meth)acrylate, and adamantyl (meth)acrylate), aromatic (meth)acrylate compounds (e.g., fluorenyl (meth)acrylate, benzyl (meth)acrylate, phenyl (meth)acrylate, o-phenylphenol (meth)acrylate, m-phenylphenol (meth)acrylate, p-phenylphenol (meth)acrylate, pentabromobenzyl (meth)acrylate, pentachlorophenyl (meth)acrylate, naphthyl (meth)acrylate, anthracenyl (meth)acrylate, and anthracene methyl (meth)acrylate), and heterocyclic (meth)acrylate compounds (e.g., piperidinyl (meth)acrylate, tetramethylpiperidinyl (meth)acrylate, and pentamethylpiperidinyl (meth)acrylate);

(meth)acrylamide compounds such as aliphatic (meth)acrylamide compounds (e.g., N-s-butyl(meth)acrylamide, N-t-butyl(meth)acrylamide, isopropylacrylamide, isohexyl(meth)acrylamide, N-t-octyl(meth)acrylamide, isooctyl(meth)acrylamide, N,N-dimethyl(meth)acrylamide, N,N-diethyl(meth)acrylamide, N,N-dibutyl(meth)acrylamide, N-methyl-N-butylacrylamide, N,N-diisopropyl(meth)acrylamide, and dimethylaminopropylacrylamide), aromatic (meth)acrylamide compounds (e.g., N-methyl-N-phenyl(meth)acrylamide), heterocyclic (meth)acrylamide compounds (e.g., N-(meth)acryloylmorpholine, N-piperidinylacrylamide, N-tetramethylpiperidinylacrylamide, N-2-methylpiperidinylacrylamide, N-3-methylpiperidinylacrylamide, and N-4-methylpiperidinylacrylamide), and (meth)acrylamide; and

mono-N-vinyl derivatives such as N-vinylimidazole, N-vinylpyrrolidone, N-vinylcaprolactam, and N-vinylcarbazole.

Other examples include N-(meth)acryloylcarbazole, (meth)acrylic acid, a potassium salt of (meth)acrylic acid, a magnesium salt of (meth)acrylic acid, and a zinc salt of (meth)acrylic acid.

When the monofunctional polymerizable compound (a1) contains a structure such as an alicyclic hydrocarbon group, an aromatic hydrocarbon, a quaternary hydrocarbon group, a heterocyclic group, or a polar group, the glass transition temperature tends to increase with the effect of the rigid backbone or polar group increasing the intermolecular restraining force of the polymer. This improves the strength of the cured product. The polymer glass transition temperature of the monofunctional polymerizable compound (a1) is preferably 80°C or more, more preferably 100°C or more. The polymer glass transition temperature may be, for example, 250°C or less, though the upper limit is not particularly limited. The alicyclic hydrocarbon group may be a monocyclic alicyclic hydrocarbon group, or a polycyclic alicyclic hydrocarbon group. Examples of the alicyclic hydrocarbon group include a cycloalkyl group such as a cyclopentyl group, a cyclohexyl group, a cycloheptyl group, or a cyclooctyl group; and a polycyclic alicyclic hydrocarbon group such as a decahydronaphthyl group, an adamantyl group, or a norbornyl group. Examples of the aromatic hydrocarbon group include an aryl group such as a phenyl group, a naphthyl group, an anthryl group, a biphenyl group, or a phenanthryl group. Examples of the heterocyclic group include a hetero five-membered ring group having one heteroatom (e.g., a pyrrolidine ring); a hetero six-membered ring group having one heteroatom (e.g., a piperidine ring, a pyran ring, a pyridine ring); a hetero five-membered ring group having two heteroatoms (e.g., an imidazolidine ring); a hetero six-membered ring group having two heteroatoms (e.g., a piperazine ring, a pyridazine ring, a pyrazine ring, a pyrimidine ring); and a hetero six-membered ring group having three heteroatoms (for example, a triazine ring such as a 1,2,3-triazine ring, a 1,2,4-triazine ring, or a 1,3,5-triazine ring).

In view of high glass transition temperature, preferred as the monofunctional polymerizable compound (a1) are (meth)acrylate compounds such as methyl methacrylate, t-butyl (meth)acrylate, 4-t-butylcyclohexyl (meth)acrylate, cyclohexyl (meth)acrylate, isobornyl (meth)acrylate, adamantyl (meth)acrylate, fluorenyl (meth)acrylate, naphthyl (meth)acrylate, anthracenyl (meth)acrylate, anthracene methyl (meth)acrylate, and pentamethylpiperidinyl (meth)acrylate; heterocyclic (meth)acrylamide compounds such as N-(meth)acryloylmorpholine; (meth)acrylamide compounds such as N-t-butyl(meth)acrylamide, N-t-octyl(meth)acrylamide, N,N-dimethyl(meth)acrylamide, N,N-diethyl(meth)acrylamide, N-piperidinylacrylamide, N-tetramethylpiperidinylacrylamide, N-2-methylpiperidinylacrylamide, N-3-methylpiperidinylacrylamide, N-4-methylpiperidinylacrylamide, and (meth)acrylamide; and a zinc salt of N-(meth)acrylic acid. More preferred are methyl methacrylate, (meth)acrylamide, N,N-diethyl(meth)acrylamide, N-t-butyl(meth)acrylamide, pentamethylpiperidinyl (meth)acrylate, N-piperidinylacrylamide, N-4-methylpiperidinylacrylamide, and a zinc salt of (meth)acrylic acid.

In view of particularly superior water resistance, preferred as the monofunctional polymerizable compound (a1) are (meth)acrylate compounds such as methyl methacrylate, t-butyl (meth)acrylate, 4-t-butylcyclohexyl (meth)acrylate,

cyclohexyl (meth)acrylate, isobornyl (meth)acrylate, adamantyl (meth)acrylate, fluorenyl (meth)acrylate, benzyl (meth)acrylate, phenyl (meth)acrylate, o-phenylphenol (meth)acrylate, naphthyl (meth)acrylate, anthracenyl (meth)acrylate, and anthracene methyl (meth)acrylate; (meth)acrylamide compounds such as N-t-butyl(meth)acrylamide, N-t-octyl(meth)acrylamide, N-methyl-N-phenyl(meth)acrylamide, and (meth)acrylamide; and N-(meth)acryloylcarbazole, N-vinylimidazole, and N-vinylcarbazole. When the monofunctional polymerizable compound (a1) is a (meth)acrylic compound having a side chain with at most two carbon atoms, the presence of the very short side chain increases the intermolecular restraining force of the polymer, and inhibits entry of a water molecule. When the monofunctional polymerizable compound (a1) contains at least one structure selected from an aromatic hydrocarbon group, an alicyclic hydrocarbon group, and a quaternary hydrocarbon group, the intermolecular restraining force of the polymer increases, and inhibits entry of a water molecule, and a highly hydrophobic structure is exposed at the outermost layer of the coating layer formed as a cured product of the coating material (A). This helps improve water resistance.

The monofunctional polymerizable compound (a2) is not particularly limited, as long as it has a glass transition temperature of less than 60°C. Examples of the monofunctional polymerizable compound (a2) include:

aliphatic (meth)acrylate compounds such as propyl (meth)acrylate, isopropyl (meth)acrylate, n-butyl (meth)acrylate, s-butyl (meth)acrylate, 2,3-dibromopropyl (meth)acrylate, 2-hydroxypropyl (meth)acrylate, 3-hydroxypropyl (meth)acrylate, butyl (meth)acrylate, 4-hydroxybutyl (meth)acrylate, pentyl (meth)acrylate, hexyl (meth)acrylate, 6-hydroxyhexyl (meth)acrylate, 10-hydroxydecyl (meth)acrylate, 2-propylheptylacrylate, lauryl (meth)acrylate, cetyl (meth)acrylate, stearyl (meth)acrylate, propylene glycol mono(meth)acrylate, glycerol mono(meth)acrylate, and erythritol mono(meth)acrylate;
aliphatic (meth)acrylamide compounds such as N-octylacrylamide, isononyl(meth)acrylamide, isodecyl(meth)acrylamide, and N-octadecyl(meth)acrylamide;
aromatic (meth)acrylate compounds (preferably, aromatic (meth)acrylate compounds having two aromatic rings) such as o-phenylphenoxymethyl acrylate, m-phenylphenoxymethyl acrylate, p-phenylphenoxymethyl acrylate, o-phenylphenoxyethyl acrylate (or ethoxylated-o-phenylphenol acrylate as it is also called), m-phenylphenoxyethyl acrylate, p-phenylphenoxyethyl acrylate, o-phenylphenoxypropyl acrylate, m-phenylphenoxypropyl acrylate, p-phenylphenoxypropyl acrylate, o-phenylphenoxybutyl acrylate, m-phenylphenoxybutyl acrylate, p-phenylphenoxybutyl acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, and 6-(p-phenoxyphenyl)hexyl (meth)acrylate;
fluorine-containing aliphatic (meth)acrylate compounds such as 2,2,2-trifluoroethyl (meth)acrylate, 1H,1 H,2H,2H-heptadecafluorodecyl (meth)acrylate, 2,2,3,3,4,4,5,5,6,6,7,7-dodecafluoroheptyl (meth)acrylate, and 1H,1H,5H-octafluoropentyl (meth)acrylate;
silane compounds such as 3-(meth)acryloyloxypropyltrimethoxysilane, and 11-(meth)acryloyloxyundecyltrimethoxysilane;
phosphorus compounds such as 2-(meth)acryloyloxyethylphosphorylcholine;
betaine monofunctional polymerizable compounds such as N-(meth)acryloyloxymethyl-N,N-dimethylammonium-α-N-methyl sulfobetaine, N-(meth)acryloyloxymethyl-N,N-dimethylammonium-α-N-ethyl sulfobetaine, N-(meth)acryloyloxymethyl-N,N-diethylammonium-α-N-methyl sulfobetaine, N-(meth)acryloyloxyethyl-N,N-dimethylammonium-α-N-methyl sulfobetaine, N-(meth)acryloyloxyethyl-N,N-diethylammonium-α-N-methyl sulfobetaine, N-(meth)acryloyloxyethyl-N,N-dimethylammonium-α-N-propyl sulfobetaine, N-(meth)acryloyloxyethyl-N,N-diethylammonium-α-N-ethyl sulfobetaine, N-(meth)acryloyloxyalkyl-N,N-dialkylammonium-α-N-alkyl carboxybetaine, N-(meth)acryloyloxymethyl-N,N-dimethylammonium-a-N-methyl carboxybetaine, N-(meth)acryloyloxymethyl-N,N-dimethylammonium-a-N-ethyl carboxybetaine, N-(meth)acryloyloxymethyl-N,N-diethylammonium-α-N-methyl carboxybetaine, N-(meth)acryloyloxyethyl-N,N-dimethylammonium-α-N-methyl carboxybetaine, N-(meth)acryloyloxyethyl-N,N-diethylammonium-α-N-methyl carboxybetaine, N-(meth)acryloyloxyethyl-N,N-dimethylammonium-α-N-ethyl carboxybetaine, N-(meth)acryloyloxymethyl-N,N-diethylammonium-α-N-ethyl carboxybetaine, N-(meth)acryloylaminoethyl-N,N-dimethylammonium-α-N-butyl sulfobetaine, and N-(meth)acryloylaminopropyl-N,N-dimethylammonium-α-N-butyl sulfobetaine; and
monomers presented in WO2006/016649, 2-(meth)acryloyloxyethyltrimethylammonium chloride, 2-(meth)acryloyloxyethyltrimethylammonium bromide, 12-(meth)acryloyloxydodecylpyridinium chloride, 12-(meth)acryloyloxydodecylpyridinium bromide, 2-(meth)acryloyloxyethyldimethyloctylammonium chloride, and 2-(meth)acryloyloxyethyldimethyloctylammonium bromide.

When the monofunctional polymerizable compound (a2) contains a structure such as a chain hydrocarbon group having 3 or more carbon atoms, an alkylene glycol chain, a fluoro group, a silyl group, a zwitterionic group, or a quaternary ammonium salt group, the glass transition temperature decreases by the effect of steric hindrance or low polar group decreasing the intermolecular restraining force of the polymer. This imparts flexibility to the cured product. The polymer glass transition temperature of the monofunctional polymerizable compound (a2) is preferably 45°C or less, more preferably 30°C or less. The polymer glass transition temperature may be, for example, -80°C or higher, though the lower limit is not particularly limited.

In view of particularly superior water resistance, preferred as the monofunctional polymerizable compound (a2) are 2-propylheptyl acrylate, lauryl (meth)acrylate, cetyl (meth)acrylate, stearyl (meth)acrylate, N-octylacrylamide, isononyl(meth)acrylamide, isodecyl(meth)acrylamide, N-octadecyl(meth)acrylamide, o-phenylphenoxymethyl (meth)acrylate, m-phenylphenoxymethyl (meth)acrylate, p-phenylphenoxymethyl (meth)acrylate, o-phenylphenoxyethyl (meth)acrylate, m-phenylphenoxyethyl (meth)acrylate, p-phenylphenoxyethyl (meth)acrylate, o-phenylphenoxypropyl (meth)acrylate, m-phenylphenoxypropyl (meth)acrylate, p-phenylphenoxypropyl (meth)acrylate, o-phenylphenoxybutyl (meth)acrylate, m-phenylphenoxybutyl (meth)acrylate, p-phenylphenoxybutyl (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, 6-(p-phenoxyphenyl)hexyl (meth)acrylate, 2,2,2-trifluoroethyl (meth)acrylate, 1H,1 H,2H,2H-heptadecafluorodecyl (meth)acrylate, 2,2,3,3,4,4,5,5,6,6,7,7-dodecafluoroheptyl (meth)acrylate, 1H,1H,5H-octafluoropentyl (meth)acrylate, 3-(meth)acryloyloxypropyltrimethoxysilane, and 11-(meth)acryloyloxyundecyltrimethoxysilane. When the monofunctional polymerizable compound (a2) contains at least one structure selected from a chain alkyl group having 8 or more carbon atoms, an aromatic hydrocarbon group, a fluoro group, and a silyl group, water resistance can more easily improve with a highly hydrophobic structure exposed at the outermost layer of the coating layer formed as a cured product of the coating material (A).

In view of high curability, preferred are 2-propylheptyl acrylate, lauryl (meth)acrylate, N-octylacrylamide, isononyl(meth)acrylamide, o-phenylphenoxymethyl (meth)acrylate, m-phenylphenoxymethyl (meth)acrylate, p-phenylphenoxymethyl (meth)acrylate, o-phenylphenoxyethyl (meth)acrylate, m-phenylphenoxyethyl (meth)acrylate, p-phenylphenoxyethyl (meth)acrylate, o-phenylphenoxypropyl (meth)acrylate, m-phenylphenoxypropyl (meth)acrylate, p-phenylphenoxypropyl (meth)acrylate, o-phenylphenoxybutyl (meth)acrylate, m-phenylphenoxybutyl (meth)acrylate, p-phenylphenoxybutyl (meth)acrylate, o-phenoxybenzyl (meth)acrylate, m-phenoxybenzyl (meth)acrylate, and p-phenoxybenzyl (meth)acrylate. In view of high hydrophobicity, preferred are 1H,1H,2H,2H-heptadecafluorodecyl (meth)acrylate, 2,2,3,3,4,4,5,5,6,6,7,7-dodecafluoroheptyl (meth)acrylate, and 1H,1H,5H-octafluoropentyl (meth)acrylate.

In view of particularly superior antifouling properties and antimicrobial properties, preferred as the monofunctional polymerizable compound (a2) are 2,2,2-trifluoroethyl (meth)acrylate, 1H,1H,2H,2H-heptadecafluorodecyl (meth)acrylate, 2,2,3,3,4,4,5,5,6,6,7,7-dodecafluoroheptyl (meth)acrylate, 1H,1H,5H-octafluoropentyl (meth)acrylate, 2-(meth)acryloyloxyethylphosphorylcholine, betaine monofunctional polymerizable compounds (such as N-(meth)acryloyloxyalkyl-N,N-dialkylammonium-α-N-alkyl sulfobetaine, N-(meth)acryloyloxymethyl-N,N-dimethylammonium-a-N-methyl sulfobetaine, N-(meth)acryloyloxymethyl-N,N-dimethylammonium-a-N-ethyl sulfobetaine, N-(meth)acryloyloxymethyl-N,N-diethylammonium-α-N-methyl sulfobetaine, N-(meth)acryloyloxyethyl-N,N-dimethylammonium-α-N-methyl sulfobetaine, N-(meth)acryloyloxyethyl-N,N-diethylammonium-α-N-methyl sulfobetaine, N-(meth)acryloyloxyethyl-N,N-dimethylammonium-α-N-ethyl sulfobetaine, N-(meth)acryloyloxymethyl-N,N-diethylammonium-α-N-ethyl sulfobetaine, N-(meth)acryloyloxyethyl-N,N-diethylammonium-α-N-ethyl sulfobetaine, N-(meth)acryloyloxyalkyl-N,N-dialkylammonium-α-N-alkyl carboxybetaine, N-(meth)acryloyloxymethyl-N,N-dimethylammonium-a-N-methyl carboxybetaine, N-(meth)acryloyloxymethyl-N,N-dimethylammonium-a-N-ethyl carboxybetaine, N-(meth)acryloyloxymethyl-N,N-diethylammonium-α-N-methyl carboxybetaine, N-(meth)acryloyloxyethyl-N,N-dimethylammonium-α-N-methyl carboxybetaine, N-(meth)acryloyloxyethyl-N,N-diethylammonium-α-N-methyl carboxybetaine, N-(meth)acryloyloxyethyl-N,N-dimethylammonium-α-N-ethyl carboxybetaine, N-(meth)acryloyloxymethyl-N,N-diethylammonium-α-N-ethyl carboxybetaine, N-(meth)acryloyloxyethyl-N,N-diethylammonium-α-N-ethyl carboxybetaine, N-(meth)acryloylaminoethyl-N,N-dimethylammonium-α-N-butyl sulfobetaine, and N-(meth)acryloylaminopropyl-N,N-dimethylammonium-α-N-butyl sulfobetaine), and monomers presented in WO2006/016649, 2-(meth)acryloyloxyethyltrimethylammonium chloride, 2-(meth)acryloyloxyethyltrimethylammonium bromide, 12-(meth)acryloyloxydodecylpyridinium chloride, 12-(meth)acryloyloxydodecylpyridinium bromide, 2-(meth)acryloyloxyethyldimethyloctylammonium chloride, and 2-(meth)acryloyloxyethyldimethyloctylammonium bromide.

When the monofunctional polymerizable compound (a2) contains a fluoro group, a highly hydrophobic structure is exposed at the outermost layer of the coating layer formed as a cured product of the coating material (A). This makes

it possible to inhibit adhesion of stains and bacteria. When the monofunctional polymerizable compound (a2) contains a zwitterionic group, a superhydrophilic structure is exposed at the outermost layer of the coating layer formed as a cured product of the coating material (A). This makes it possible to easily wash away stains and bacteria adhering to the coating, in addition to inhibiting adhesion of stains and bacteria. When the monofunctional polymerizable compound (a2) contains a quaternary ammonium salt group, growth of bacteria can be inhibited.

In view of easy availability, preferred are 2-(meth)acryloyloxyethylphosphorylcholine, N-methacryloyloxyethyl-N,N-dimethylammonium-α-N-propyl sulfobetaine, N-methacryloyloxyethyl-N,N-dimethylammonium-α-N-ethyl carboxybetaine, N-methacryloyloxyethyl-N,N-dimethylammonium-α-N-methyl carboxybetaine, 3-(meth)acryloylaminopropyl-N,N-dimethylammonium-α-N-ethyl carboxybetaine, 3-(meth)acryloylaminopropyl-N,N-dimethylammonium-α-N-butyl sulfobetaine, 2-(meth)acryloyloxyethyltrimethylammonium chloride, 12-(meth)acryloyloxydodecylpyridinium bromide, and 2-(meth)acryloyloxyethyldimethyloctylammonium chloride.

The monofunctional polymerizable compound (a) may be used alone, or two or more thereof may be used in combination.

Polyfunctional Polymerizable Compound (b)

The polyfunctional polymerizable compound (b) has a Mw/n of 120 or more, where Mw is the molecular weight, and n is the number of polymerizable groups per molecule. In an energy ray-curable coating material (A) for three-dimensional shaped articles of the present invention, the polyfunctional polymerizable compound (b) is used to impart toughness to a cured product of the coating material (A). When the polyfunctional polymerizable compound has a low molecular weight (for example, less than 150), and/or has polymerizable groups in excess, the cured product becomes very brittle as a result of large shrinkage occurring during cure. This is because of the crosslinkage of short chains impairing the fluidity of the molecular chain and making it difficult to relieve the shrinkage stress or strain. With a polyfunctional polymerizable compound having a Mw/n of 120 or more, the molecular chain retains some fluidity because of crosslinking of relatively long chains. Preferably, Mw/n is 200 or more, more preferably 300 or more, even more preferably 400 or more. A polyfunctional polymerizable compound having a Mw/n of 500 or more is particularly preferred because such a polyfunctional polymerizable compound has an increased molecular weight between crosslinking points, and can impart toughness while providing superior flexibility and reducing shrinkage. In view of curability, Mw/n is preferably 50,000 or less. Here, Mw means the molecular weight (a sum of the atomic weights present in the molecule) when the polyfunctional polymerizable compound (b) is a compound that does not have a main-chain structure with two or more of the same or several kinds of repeating units such as that of an oligomer, that is, a compound having no polymer backbone (for example, a polyfunctional polymerizable compound (b1-1) having no polymer backbone; described later). When the polyfunctional polymerizable compound (b) is a compound having a polymer backbone (for example, a polyfunctional polymerizable compound (b1-2) having a polymer backbone; described later), Mw/n means a double bond equivalent (a molecular weight divided by the number of the double bonds in the same molecule).

The value of double bond equivalent can be estimated from, for example, the weight-average molecular weight of a specimen, and the amount of double bonds quantified in the specimen based on an iodine value measured by the method of JIS K 0070:1992. In the present invention, "weight-average molecular weight" means a weight-average molecular weight determined in terms of polystyrene by gel permeation chromatography.

The polyfunctional polymerizable compound (b) includes a polyfunctional polymerizable compound (b1) having a polymer glass transition temperature of 60°C or more (hereinafter, also referred to simply as "polyfunctional polymerizable compound (b1)"), and a polyfunctional polymerizable compound (b2) having a polymer glass transition temperature of less than 60°C (hereinafter, also referred to simply as "polyfunctional polymerizable compound (b2)"). In view of the toughness of a polymer of the polyfunctional polymerizable compound (b) constituting a cured product of the coating material (A), it is preferable to use the polyfunctional polymerizable compound (b1) and the polyfunctional polymerizable compound (b2) in combination.

The polyfunctional polymerizable compound (b1) is not particularly limited, as long as it has a glass transition temperature of 60°C or more. Examples of the polyfunctional polymerizable compound (b1) include a polyfunctional polymerizable compound (b1-1) having no polymer backbone, and a polyfunctional polymerizable compound (b1-2) having a polymer backbone. Here, "polymer backbone" refers to a main-chain structure having two or more of the same or several kinds of repeating units. The polymer glass transition temperature of the polyfunctional polymerizable compound (b1) is preferably 80°C or more, more preferably 100°C or more. The polymer glass transition temperature may be, for example, 300°C or less, though the upper limit is not particularly limited.

Specific examples of the polyfunctional polymerizable compound (b1-1) having no polymer backbone (hereinafter, also referred to as "polyfunctional polymerizable compound (b1-1)") include aromatic polyfunctional polymerizable compounds, aliphatic polyfunctional polymerizable compounds, alicyclic polyfunctional polymerizable compounds, and nitrogen ring-containing polyfunctional polymerizable compounds.

Specific examples of the aromatic polyfunctional polymerizable compounds include 2,2-bis((meth)acryloyloxyphenyl)propane, 2,2-bis[4-(3-acryloyloxy)-2-hydroxypropoxyphenyl]propane, 2,2-bis[4-(3-methacryloyloxy)-2-hydroxypropoxyphenyl]propane (commonly known as "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate.

Specific examples of the aliphatic polyfunctional polymerizable compounds include bifunctional polymerizable compounds, for example, such as triethylene glycol di(meth)acrylate, polyethylene glycol di(meth)acrylate, dipropylene glycol di(meth)acrylate, polypropylene glycol di(meth)acrylate, dibutylene glycol di(meth)acrylate, neopentyl glycol dimethacrylate, 1,6-hexanediol dimethacrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, 2,2,4-trimethylhexamethylene bis(2-carbamoyloxyethyl)dimethacrylate (commonly known as "UDMA"), N,N-(2,2,4-trimethylhexamethylene)bis[2-(aminocarboxy)propane-1,3-diol]tetra(meth)acr ylate, phenyl glycidyl ether acrylate hexamethylene diisocyanate urethane prepolymer, and pentaerythritol triacrylate hexamethylene diisocyanate urethane prepolymer. Examples of tri- and higher-functional polymerizable compounds include 1,7-diacryloyloxy-2,2,6,6-tetra(meth)acryloyloxymethyl-4-oxyheptane, pentaerythritol triacrylate toluene diisocyanate urethane prepolymer, pentaerythritol triacrylate isophorone diisocyanate urethane prepolymer, and dipentaerythritol pentaacrylate hexamethylene diisocyanate urethane prepolymer.

Specific examples of the alicyclic polyfunctional polymerizable compounds include cyclohexyl di(meth)acrylate, isobornyl di(meth)acrylate, tricyclodecane dimethanol di(meth)acrylate, adamantyl di(meth)acrylate, and fluorenyl di(meth)acrylate.

Specific examples of the nitrogen ring-containing polyfunctional polymerizable compounds include triallyl cyanurate, triallyl isocyanurate, and tris(2-acryloyloxyethyl)isocyanurate.

Because of the absence of a polymer backbone, these compounds do not have an overly large molecular weight between crosslinking points. This makes it easier to physically increase the intermolecular restraining force, particularly by covalent bonding, and the strength of the cured product improves.

Specific examples of the polyfunctional polymerizable compound (b1-2) having a polymer backbone include polyacrylates, polyurethanes, polyesters, polyethers, and polysilsesquioxanes, or urethanized (meth)acrylate compounds and aliphatic urethane (meth)acrylates formed by introducing a urethane bond to these compounds.

Specific examples of polyacrylates that can be used as the polyfunctional polymerizable compound (b1-2) having a polymer backbone include ACRIT BR-600, ACRIT BR-930MB, ACRIT 8KX-212, and ACRIT 8KQ-2001 manufactured by Taisei Fine Chemical Co,. Ltd., Art Cure RA-3704MB manufactured by Negami Chemical Industrial Co., Ltd., Art Cure RA-3953MB manufactured by Negami Chemical Industrial Co., Ltd., Art Cure RA-4101 manufactured by Negami Chemical Industrial Co., Ltd., Art Cure OAP-5000 manufactured by Negami Chemical Industrial Co., Ltd., Art Cure OAP-2511 manufactured by Negami Chemical Industrial Co., Ltd., Art Cure AHC-9202MI80 manufactured by Negami Chemical Industrial Co., Ltd., and EBECRYL 305 manufactured by Daicel allnex. Specific examples of the polyurethanes include radical polymerizable group-containing urethane prepolymers presented in JP 2012-72327 A, KRM8191 manufactured by Daicel allnex, EBECRYL 4738 manufactured by Daicel allnex, and ACRIT BUH-1094, ACRIT BUH-4005A, ACRIT BUH-4025A, ACRIT BUX-015A, and ACRIT BUX-047A manufactured by Taisei Fine Chemical Co,. Ltd. Specific examples of the polyesters include EBECRYL 812 and EBECRYL 1830 manufactured by Daicel allnex, and EBECRYL 851 manufactured by Daicel allnex. Specific examples of the polyethers include CN983 NS, CN985, and CN989 NS manufactured by Arkema. Specific examples of the polysilsesquioxanes include AC-SQ TA-100, MAC-SQ TM-100, AC-SQ SI-20, MAC-SQ SI-20, and MAC-SQ HDM manufactured by Toagosei Co., Ltd., and SP-6120(H2O) and SP-6120 (MEK) manufactured by Konishi Chemical Ind. Co., Ltd. Examples of the aliphatic urethane (meth)acrylates include EBECRYL 1258 and EBECRYL 1291 manufactured by Daicel allnex.

In view of particularly superior water resistance, preferred as the polyfunctional polymerizable compound (b1) are 2,2,4-trimethylhexamethylene bis(2-carbamoyloxyethyl)dimethacrylate, N,N-(2,2,4-trimethylhexamethylene)bis[2-(aminocarboxy)propane-1,3-diol]tetra(meth)acr ylate, phenyl glycidyl ether acrylate hexamethylene diisocyanate urethane prepolymer, KRM8191 manufactured by Daicel allnex, ACRIT BR-600 and ACRIT BR-930MB manufactured by Taisei Fine Chemical Co,. Ltd., and AC-SQ TA-100, MAC-SQ TM-100, AC-SQ SI-20, MAC-SQ SI-20, and MAC-SQ HDM manufactured by Toagosei Co., Ltd. When the polyfunctional polymerizable compound (b1) has a high polarity unit, such as a urethane bond, on the main chain, the intermolecular restraining force of the polymer increases, and inhibits entry of a water molecule. When the main chain or a side chain of the polyfunctional polymerizable compound (b1) contains at least one structure selected from an aromatic hydrocarbon group, an alicyclic hydrocarbon group, and a quaternary hydrocarbon group, the intermolecular restraining force of the polymer increases, and inhibits entry of a

water molecule, and a highly hydrophobic structure is exposed at the outermost layer of the coating layer formed as a cured product of the coating material (A). This helps improve water resistance.

The polyfunctional polymerizable compound (b2) is not particularly limited, as long as it has a glass transition temperature of less than 60°C. Examples of the polyfunctional polymerizable compound (b2) include polyacrylates, polyurethanes, polyesters, polyethers, polyglycerin, and polybutadiene, or urethanized (meth)acrylic compounds, aromatic urethane (meth)acrylates, and aliphatic urethane (meth)acrylates formed by introducing a urethane bond to these compounds. The polymer glass transition temperature of the polyfunctional polymerizable compound (b2) is preferably 45°C or less, more preferably 30°C or less. The polymer glass transition temperature may be, for example, -80°C or higher, though the lower limit is not particularly limited.

Examples of the polyacrylates include ACRIT 8KX-078, ACRIT 8BS-9000, ACRIT 8WX-018, ACRIT 8WX-030, ACRIT 1WX-049, ACRIT 1WX-1020, ACRIT 8SS-723, and ACRIT 8FS-001 manufactured by Taisei Fine Chemical Co,. Ltd., and RA-331MB, RA-341, MAP-4000, and MAP-2506 manufactured by Negami Chemical Industrial Co., Ltd. Specific examples of the polyurethanes include 8UH-1094, 8UH-4005A, and 8UH-4025A manufactured by Taisei Fine Chemical Co,. Ltd., and UF-8001G manufactured by Kyoeisha Chemical Co., Ltd. Specific examples of the polyesters include EBECRYL 450, EBECRYL 810, EBECRYL 870, EBECRYL 884, and EBECRYL 885 manufactured by Daicel allnex. Specific examples of the polyethers include UF-0146, UF-07DF, UF-A01P, UF-C01, UF-C02, UF-C03, and UF-C04 manufactured by Kyoeisha Chemical Co., Ltd., EBECRYL 80 manufactured by Daicel allnex, and CN996 NS and CN9004 manufactured by Arkema. Specific examples of the polyglycerin include SA-TE6, SA-TE12, and SA-TE60 manufactured by Sakamoto Yakuhin Kogyo Co., Ltd.. Specific examples of the polybutadiene include BAC-45 manufactured by Osaka Organic Chemical Industry Ltd.. Examples of the aromatic urethane (meth)acrylate include EBECRYL 4501 manufactured by Daicel allnex. Examples of the aliphatic urethane (meth)acrylate include EBECRYL 8465, EBECRYL 8807, EBECRYL 4101, and EBECRYL 4201 manufactured by Daicel allnex. As another example, the polyfunctional polymerizable compound (b2) may be LIPIDURE-CR2001 manufactured by NOF Corporation.

The urethanized (meth)acrylic compound as a polyfunctional polymerizable compound (b2) is a compound having incorporated therein a (meth)acryl group and a urethane bond. An example of such a compound is a urethanized (meth)acrylic compound having at least one structure selected from the group consisting of a polyester, a polycarbonate, a polyurethane, a polyether, a poly-conjugated diene, and a hydrogenated poly-conjugated diene (hereinafter, these are also referred to as "polymer backbones"), per molecule.

Specific examples of the polymer backbone-containing urethanized (meth)acrylic compound as a polyfunctional polymerizable compound (b2) include urethanized (meth)acrylic compounds presented in WO2018/038056. The urethanized (meth)acrylic compound can be easily synthesized by, for example, an addition reaction of a polyol containing the polymer backbone, a compound having an isocyanate group (-NCO), and a (meth)acrylic compound having a hydroxyl group (-OH). Alternatively, the urethanized (meth)acrylic compound can be synthesized with ease by, for example, allowing lactone or alkylene oxide to undergo a ring-opening addition reaction with a (meth)acrylic compound having a hydroxyl group, and causing the resulting compound having a terminal hydroxyl group to undergo an addition reaction with a compound having an isocyanate group.

The polyol containing the polymer backbone is not particularly limited, as long as it has the above structure. Examples of the polyester include: a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of β-propiolactone, a polymer of γ-butyrolactone, a polymer of δ-valerolactone, a polymer of ε-caprolactone, and a copolymer of these. Examples of the polycarbonate include a polycarbonate derived from an aliphatic diol having 2 to 12 carbon atoms, a polycarbonate derived from bisphenol A, and a polycarbonate derived from a C2 to C12 aliphatic diol and bisphenol A. Examples of the polyurethane include a polymer of a C2 to C12 aliphatic diol and a C1 to C12 diisocyanate. Examples of the polyether include polyethylene glycol, polypropylene glycol, polybutylene glycol, and poly(1-methylbutylene glycol). Examples of the poly-conjugated diene and the hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenation products of these.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxyl group-containing (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxyl group-containing (meth)acrylamide compounds such as N-hydroxyethyl(meth)acrylamide, and N,N-bis(2-hydroxyethyl)(meth)acrylamide.

The addition reaction of the compound having an isocyanate group and the (meth)acrylic compound having a hydroxyl group can be performed following a known method, and the method is not particularly limited.

The polyfunctional polymerizable compound as a urethanized (meth)acrylic compound obtained by the foregoing method is, for example, a product of a reaction of any combination of: a polyol having at least one structure selected from the group consisting of a polyester, a polycarbonate, a polyurethane, a polyether, a poly-conjugated diene, and a hydrogenated poly-conjugated diene; a compound having an isocyanate group; and a (meth)acrylic compound having a hydroxyl group.

In view of viscosity and strength, the weight-average molecular weight of the urethanized (meth)acrylic compound having a polymer backbone is preferably 500 to 50,000, more preferably 750 to 30,000, even more preferably 1,000 to 15,000.

In view of particularly superior water resistance, preferred as the polyfunctional polymerizable compound (b2) are ACRIT 8FS-001, ACRIT BS-9000, and ACRIT 8SS-723 manufactured by Taisei Fine Chemical Co,. Ltd. When the main chain or a side chain of the polyfunctional polymerizable compound (b2) contains at least one structure selected from an aromatic hydrocarbon group, an alicyclic hydrocarbon group, a quaternary hydrocarbon group, a fluoro group, and a silyl group, the intermolecular restraining force of the polymer increases, and inhibits entry of a water molecule, and a highly hydrophobic structure is exposed at the outermost layer of the coating layer formed as a cured product of the coating material (A). This helps improve water resistance.

In view of superior curability, more preferred as the polyfunctional polymerizable compound (b2) are ACRIT 8FS-001, ACRIT BS-9000, and ACRIT 8SS-723 manufactured by Taisei Fine Chemical Co,. Ltd.

In view of particularly superior antifouling properties and antimicrobial properties, preferred as the polyfunctional polymerizable compound (b2) are ACRIT 8FS-001 manufactured by Taisei Fine Chemical Co,. Ltd., LIPIDURE-CR2001 manufactured by NOF Corporation, and ACRIT 8WX-018, ACRIT 8WX-030, ACRIT 1WX-049, and ACRIT 1WX-1020 manufactured by Taisei Fine Chemical Co,. Ltd. When the polyfunctional polymerizable compound (b2) contains a fluoro group on a side chain, a highly hydrophobic structure is exposed at the outermost layer of the coating layer formed as a cured product of the coating material (A). This makes it possible to inhibit adhesion of stains and bacteria. When the polyfunctional polymerizable compound (b2) contains a zwitterionic group on a side chain, a superhydrophilic structure is exposed at the outermost layer of the coating layer formed as a cured product of the coating material (A). This makes it possible to easily wash away stains and bacteria adhering to the coating, in addition to inhibiting adhesion of stains and bacteria. When the polyfunctional polymerizable compound (b2) contains a quaternary ammonium salt group on a side chain, growth of bacteria can be inhibited. A certain preferred embodiment is, for example, an energy ray-curable coating material (A) for three-dimensional shaped articles in which the monofunctional polymerizable compound (a) (preferably, the monofunctional polymerizable compound (a2)) and/or the polyfunctional polymerizable compound (b) (preferably, the polyfunctional polymerizable compound (b2)) have at least one selected from an alicyclic hydrocarbon group, an aromatic hydrocarbon group, a chain alkyl group having 8 or more carbon atoms, a fluoro group, and a silyl group. Another preferred embodiment is, for example, an energy ray-curable coating material (A) for three-dimensional shaped articles in which the monofunctional polymerizable compound (a) and/or the polyfunctional polymerizable compound (b) have a zwitterionic group and/or a quaternary ammonium salt group.

The polyfunctional polymerizable compound (b) may be used alone, or two or more thereof may be used in combination.

In view of superior toughness, it is preferable that the polymerizable compounds be a combination of the monofunctional polymerizable compound (a1) having a polymer glass transition temperature of 60°C or more, the polyfunctional polymerizable compound (b2) having a polymer backbone and a glass transition temperature of less than 60°C, the monofunctional polymerizable compound (a2) having a polymer glass transition temperature of less than 60°C, and the polyfunctional polymerizable compound (b1) having a polymer glass transition temperature of 60°C or more. Toughness is the property to resist fracture by virtue of flexibility while being high in strength. Accordingly, the coating layer formed as a cured product of the coating material (A) can improve toughness when at least one of the monofunctional polymerizable compound (a1) having a polymer glass transition temperature of 60°C or more, and the polyfunctional polymerizable compound (b1) having a polymer glass transition temperature of 60°C or more, both of which can improve strength, is used with at least one of the monofunctional polymerizable compound (a2) having a polymer glass transition temperature of less than 60°C, and the polyfunctional polymerizable compound (b2) having a polymer backbone and a glass transition temperature of less than 60°C, both of which can improve flexibility.

Polymerization Initiator (c)

The polymerization initiator (c) can be selected from polymerization initiators used in industry, as long as the present invention can exhibit its effects. The polymerization initiator (c) includes a photopolymerization initiator (c1) and a thermal polymerization initiator (c2). In view of the convenience of enabling coating by taking advantage of secondary polymerization of a shaped article, preferred for use is photopolymerization initiator (c1). The polymerization initiator (c)

may be used alone, or two or more thereof may be used in combination.

Specific examples of the photopolymerization initiator (c1) include: radical polymerization initiators, for example, such as ketals, α-diketones, coumarins, anthraquinones, benzoinalkyl ether compounds, α-hydroxy ketone compounds, glyoxy ester compounds, α-aminoketone compounds, (bis)acylphosphine oxides, oxime ester compounds, acridine compounds, metallocene compounds, and germanium compounds. Examples of cationic polymerization initiators include sulfonium salts, iodonium salts, phenacylsulfonium salts, hydroxyphenylsulfonium salts, sulfoxonium salts, derivatives of sulfonic acid, phosphoric acid esters, phenolsulfonic acid esters, carboxylic acid esters, aryldiazonium salts, iron arene complexes, pyridinium salts, quinolinium salts, o-nitrobenzyl group-containing compounds, diazonaphthoquinone, and N-hydroxyimidesulfonate.

In view of reducing a shade of yellow, preferred as the photopolymerization initiator (c1) are those that show little tailing in the visible light region, preferably at least one selected from the group consisting of an α-hydroxyketone compound, an α-aminoketone compound, and an iodonium salt. Particularly preferred for stability of the illuminant used to generate radicals are (bis)acylphosphine oxides and salts thereof, oxime ester compounds, and acridine compounds and metallocene compounds. It is also preferable to use at least one selected from the group consisting of a (bis)acylphosphine oxide and an α-diketone because the energy ray-curable coating material (A) for three-dimensional shaped articles can have excellent photocurability in the ultraviolet region and visible light region, and shows sufficient photocurability regardless of whether the light source used is a laser, a halogen lamp, a light emitting diode (LED), or a xenon lamp.

Examples of the α-hydroxyketone compounds include 1-hydroxycyclohexyl phenyl ketone, 2-hydroxy-2-methyl-1-phenylpropan-1-one, 1 -[4-(2-hydroxyethoxy)phenyl]-2-hydroxy-2-methyl-1 -propan-1 -one, and 2-hydroxy-1-{4[4-(2-hydroxy-2-methylpropionyl)benzyl]phenyl}-2-methylpropan-1-one. Examples of commercially available products include Omnirad 184, Omnirad 500, Omnirad 1173, and Omnirad 2959 (all manufactured by IGM Resins).

Examples of the glyoxy ester compounds include methylphenyl glyoxylate, ethylphenyl glyoxylate, 2-(2-hydroxyethoxy)ethyl oxyphenylacetate, and 2-(2-oxo-2-phenylacetoxyethoxy)ethyl oxyphenylacetate. Examples of commercially available products include Omnirad 754 and Omnirad MBF (manufactured by IGM Resins).

Examples of the α-aminoketone compounds include 2-benzyl-2-dimethylamino-1-(4-morpholinophenyl)-butan-1-one, 2-dimethylamino-2-(4-methylbenzyl)-1-(4-morpholin-4-yl-phenyl)-butan-1-one, and 2-methyl-1-(4-methylthiophenyl)-2-morpholinopropan-1-one. Examples of commercially available products include Omnirad 907, Omnirad 369, Omnirad 379, and Omnirad 1300 (manufactured by IGM Resins).

Examples of the iodonium salts include 4-isobutylphenyl-4'-methylphenyl iodonium hexafluorophosphate, bis(dodecylphenyl)iodonium hexamethoxyantimonate, 4-isopropylphenyl-4'-methylphenyl iodonium tetrakis pentamethoxyphenyl borate, and 4-isopropylphenyl-4'-methylphenyl iodonium tetrakis pentafluorophenyl borate. Examples of commercially available products include Omnicat 250 (manufactured by IGM Resins), Rhodorsil 2074 (manufactured by Solvay Japan), and IK-1 (manufactured by San-Apro Ltd.).

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, sodium salts of 2,4,6-trimethylbenzoyl phenylphosphine oxide, potassium salts of 2,4,6-trimethylbenzoyldiphenylphosphine oxide, and ammonium salts of 2,4,6-trimethylbenzoyldiphenylphosphine oxide. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Other examples include compounds presented in JP 2000-159621 A. These may be used alone, or two or more thereof may be used in combination.

Examples of the oxime ester compounds include 1-[4-(phenylthio)phenyl]octane-1,2-dione 2-(O-benzoyloxime), 1-[6-(2-methylbenzoyl)-9-ethyl-9H-carbazol-3-yl]ethanone O-acetyloxime, and 2-(acetyloxyiminomethyl)thioxanthen-9-one. Examples of commercially available products include Irgacure OXE01 and Irgacure OXE02 (both manufactured by BASF), and N-1919 (manufactured by ADEKA). These may be used alone, or two or more thereof may be used in combination. The oxime ester compounds may have a heterocyclic ring structure within the molecule. The presence of a heterocyclic ring structure provides superior absorption of light near 355 nm wavelength or near 405 nm wavelength, and improves sensitivity. The heterocyclic ring structure may be at least one selected from the group consisting of a carbazole skeleton, a xanthene skeleton, and a thioxanthone skeleton, or may be a compound having a carbazole skeleton.

Examples of the acridine compounds include:

compounds having one acridinyl group represented by the following general formula [I],

[Chem. 1]

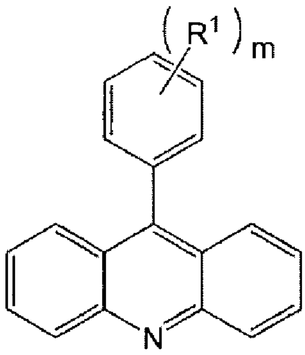

[I]

wherein $R^1$ represents a halogen atom, an amino group, a carboxy group, an alkyl group having 1 to 6 carbon atoms, an alkoxy group having 1 to 6 carbon atoms, or an alkylamino group having 1 to 6 carbon atoms, and m represents an integer of 0 to 5; and

compounds having two acridinyl groups represented by the following general formula [II],

[Chem. 2]

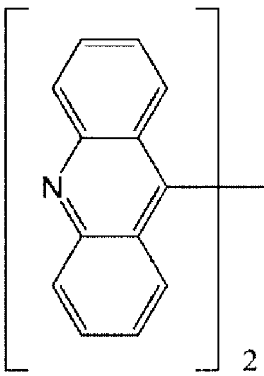

[I I]

wherein $R^2$ represents an alkylene group having 2 to 20 carbon atoms, an oxadialkylene group having 2 to 20 carbon atoms, or a thiodialkylene group having 2 to 20 carbon atoms.

Examples of acridine compounds represented by general formula [I] include 9-phenylacridine, 9-(p-methylphenyl)acridine, 9-(m-methylphenyl)acridine, 9-(p-chlorophenyl)acridine, 9-(m-chlorophenyl)acridine, 9-aminoacridine, 9-dimethylaminoacridine, 9-diethylaminoacridine, and 9-pentylaminoacridine. Examples of acridine compounds represented by general formula [II] include bis(9-acridinyl)alkanes (such as 1,2-bis(9-acridinyl)ethane, 1,3-bis(9-acridinyl)propane, 1,4-bis(9-acridinyl)butane, 1,5-bis(9-acridinyl)pentane, 1,6-bis(9-acridinyl)hexane, 1,7-bis(9-acridinyl)heptane, 1,8-bis(9-acridinyl)octane, 1,9-bis(9-acridinyl)nonane, 1,10-bis(9-acridinyl)decane, 1,11-bis(9-acridinyl)undecane, 1,12-bis(9-acridinyl)dodecane, 1,14-bis(9-acridinyl)tetradecane, 1,16-bis(9-acridinyl)hexadecane, 1,18-bis(9-acridinyl)octadecane, and 1,20-bis(9-acridinyl)eicosane), 1,3-bis(9-acridinyl)-2-oxapropane, 1,3-bis(9-acridinyl)-2-tiapropane, and 1,5-bis(9-acridinyl)-3-tiapentane. These may be used alone, or two or more thereof may be used in combination.

Examples of the metallocene compounds include bis($\eta^5$-2,4-cyclopentadien-1-yl)-bis(2,6-difluoro-3-(1H-pyrrol-1-yl)-phenyl)titanium. Examples of commercially available products include Omnirad 784 (manufactured by IGM Resins). These may be used alone, or two or more thereof may be used in combination. Examples of the germanium compounds include monoacyl germanium compounds such as benzoyltrimethylgermanium(IV); and diacyl germanium compounds such as dibenzoyldiethylgermanium and bis(4-methoxybenzoyl)-diethylgermanium.

Examples of the $\alpha$-diketones include diacetyl, benzyl, camphorquinone, 2,3-pentadione, 2,3-octadione, 9,10-phenanthrenequinone, 4,4'-oxybenzyl, and acenaphthenequinone. Camphorquinone is particularly preferred when a visible-light light source is used.

Organic peroxides and azo compounds are preferred specific examples of thermal polymerization initiator (c2). The organic peroxides and azo compounds used as the thermal polymerization initiator (c2) are not particularly limited,

and may be known compounds. Typical examples of organic peroxides include ketone peroxides, hydroperoxides, diacyl peroxides, dialkyl peroxides, peroxy ketals, peroxyesters, and peroxydicarbonates.

Examples of ketone peroxides used as the thermal polymerization initiator (c2) include methyl ethyl ketone peroxide, methyl isobutyl ketone peroxide, methyl cyclohexanone peroxide, and cyclohexanone peroxide.

Examples of hydroperoxides used as the thermal polymerization initiator (c2) include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of diacyl peroxides used as the thermal polymerization initiator (c2) include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of dialkyl peroxides used as the thermal polymerization initiator (c2) include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of peroxy ketals used as the thermal polymerization initiator (c2) include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of peroxyesters used as the thermal polymerization initiator (c2) include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxyvalerate.

Examples of peroxydicarbonates used as the thermal polymerization initiator (c2) include di-3-methoxyperoxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

Examples of azo compounds used as the thermal polymerization initiator (c2) include 2,2'-azobis(isobutyronitrile), 2,2'-azobis(2-methylbutyronitrile), 2,2'-azobis(2,4-dimethylvaleronitrile), 2,2'-azobis(4-methoxy-2,4-dimethylvaleronitrile), 1,1'-azobis(cyclohexane-1-carbonitrile), 4,4'-azobis(4-cyanovaleric acid), 2,2-azobis[2-(2-imidazolin-2-yl)propane], and 2,2'-azobis{2-methyl-N-[1,1-bis(hydroxymethyl)-2-hydroxyethyl]propionamide}.

Among these organic peroxides and azo compounds, preferred as thermal polymerization initiator (c2) are diacyl peroxides, hydroperoxides, and azo compounds for their radical generating potential in the presence of a (meth)acrylic acid ester polymer. More preferred as thermal polymerization initiator (c2) are hydroperoxides because hydroperoxides reduce discoloration of the cured product or bubble formation. Even more preferred are benzoyl peroxide, cumene hydroperoxide, t-butyl hydroperoxide, 1,1,3,3-tetramethylbutyl hydroperoxide, 2,2'-azobis(isobutyronitrile), and 2,2'-azobis(2-methylbutyronitrile). Particularly preferred is 1,3,3-tetramethylbutyl hydroperoxide.

The content of the polymerization initiator (c) in an energy ray-curable coating material (A) for three-dimensional shaped articles of the present invention is not particularly limited. However, in view of curability and other properties, the polymerization initiator (c) is preferably 0.01 to 20 parts by mass relative to total 100 parts by mass of the monofunctional polymerizable compound (a) and the polyfunctional polymerizable compound (b). When the content of polymerization initiator (c) is less than 0.01 parts by mass, polymerization may fail to sufficiently proceed, and it may not be possible to obtain a formed product. The content of polymerization initiator (c) is more preferably at least 0.05 parts by mass, even more preferably at least 0.1 parts by mass relative to total 100 parts by mass of the monofunctional polymerizable compound (a) and the polyfunctional polymerizable compound (b). When the content of polymerization initiator (c) is more than 20 parts by mass, there is a possibility of precipitation from the energy ray-curable coating material (A) for three-dimensional shaped articles when the solubility of the polymerization initiator itself is low. The content of polymerization initiator (c) is more preferably at most 15 parts by mass, even more preferably at most 10 parts by mass, particularly preferably at most 5.0 parts by mass relative to total 100 parts by mass of the monofunctional polymerizable compound (a) and the polyfunctional polymerizable compound (b).

In view of superior toughness, it is preferable that the total content of the monofunctional polymerizable compound (a1) having a polymer glass transition temperature of 60°C or more, and the polyfunctional polymerizable compound (b1) having a polymer glass transition temperature of 60°C or more be 15 to 90 parts by mass, and that the total content of the monofunctional polymerizable compound (a2) having a polymer glass transition temperature of less than 60°C, or the polyfunctional polymerizable compound (b2) having a polymer backbone and a glass transition temperature of less than 60°C be 10 to 85 parts by mass, relative to total 100 parts by mass of the monofunctional polymerizable compound (a) and the polyfunctional polymerizable compound (b). The total content of the monofunctional polymerizable compound (a1) having a polymer glass transition temperature of 60°C or more, or the polyfunctional polymerizable compound (b1) having a polymer glass transition temperature of 60°C or more is more preferably 25 to 80 parts by mass, even more preferably 35 to 70 parts by mass. The total content of the monofunctional polymerizable compound (a2) having a polymer glass transition temperature of less than 60°C, or the polyfunctional polymerizable compound

(b2) having a polymer backbone and a glass transition temperature of less than 60°C is more preferably 20 to 75 parts by mass, even more preferably 30 to 65 parts by mass.

In view of superior water resistance, the total content of the monofunctional polymerizable compound (a1) having a polymer glass transition temperature of 60°C or more, the monofunctional polymerizable compound containing an alicyclic hydrocarbon group, the monofunctional polymerizable compound containing an aromatic hydrocarbon group, the monofunctional polymerizable compound containing a tert-butyl group, the monofunctional polymerizable compound containing a chain alkyl group having 8 or more carbon atoms, the monofunctional polymerizable compound containing a fluoro group, the monofunctional polymerizable compound containing a silyl group, the polyfunctional polymerizable compound (b1-2) having a polymer backbone and a glass transition temperature of 60°C or more, the polyfunctional polymerizable compound containing a chain alkyl group having 8 or more carbon atoms, the polyfunctional polymerizable compound containing a fluoro group, and the polyfunctional polymerizable compound containing a silyl group is preferably 10 to 100 parts by mass, more preferably 20 to 100 parts by mass, even more preferably 30 to 100 parts by mass relative to total 100 parts by mass of the monofunctional polymerizable compound (a) and the polyfunctional polymerizable compound (b).

In view of superior antifouling properties and antimicrobial properties, the total content of the monofunctional polymerizable compound containing a zwitterionic group, the monofunctional polymerizable compound containing a quaternary ammonium salt group, the polyfunctional polymerizable compound containing a zwitterionic group, and the polyfunctional polymerizable compound containing a quaternary ammonium salt group is preferably 10 to 100 parts by mass, more preferably 20 to 100 parts by mass, even more preferably 30 to 100 parts by mass relative to total 100 parts by mass of the monofunctional polymerizable compound (a) and the polyfunctional polymerizable compound (b).

Preferably, an energy ray-curable coating material (A) for three-dimensional shaped articles of the present invention comprises a solvent (d) to reduce viscosity and thereby improve the uniformity of the coating layer, in addition to reducing cost, provided that it is not detrimental to the intent and purpose of the present invention. Examples of the solvent include water, methanol, ethanol, isopropyl alcohol, acetone, ethyl acetate, methyl ethyl ketone, chloroform, tetrahydrofuran, diethyl ether, diisopropyl ether, dimethylformamide, and dimethyl sulfoxide. In view of ease of removal, the solvent is preferably one having a boiling point at normal pressure of 80°C or less, more preferably 60°C or less. In view of ease of handling by a worker, the flash point is preferably -30°C or higher, more preferably -20°C or higher.

An energy ray-curable coating material (A) for three-dimensional shaped articles of the present invention is not particularly limited, as long as it comprises the monofunctional polymerizable compound (a) and/or the polyfunctional polymerizable compound (b), and the polymerization initiator (c). For example, an energy ray-curable coating material (A) for three-dimensional shaped articles of the present invention may additionally comprise components other than these. An energy ray-curable coating material (A) for three-dimensional shaped articles of the present invention can be produced according to a known method.

In order to improve curability with energy rays, an energy ray-curable coating material (A) for three-dimensional shaped articles of the present invention may comprise a polyfunctional polymerizable compound (e) having a Mw/n of less than 120, provided that it is not detrimental to the intent and purpose of the present invention. Examples of the polyfunctional polymerizable compound (e) having a Mw/n of less than 120 include 1,2-ethanediol di(meth)acrylate, 1,3-propanediol di(meth)acrylate, 1,4-butanediol di(meth)acrylate, 1,3-butanediol di(meth)acrylate, 1,5-pentanediol diacrylate, neopentyl glycol diacrylate, 1,6-hexanediol diacrylate, diethylene glycol di(meth)acrylate, glycerol di(meth)acrylate, 1-(acryloyloxy)-3-(methacryloyloxy)-2-propanol, trimethylolpropane tri(meth)acrylate, ditrimethylolpropane tetra(meth)acrylate, pentaerythritol tetra(meth)acrylate, 1,4-diglycidyloxybutane, triglycidyl isocyanurate, and N,N-methylenebisacrylamide. In view of imparting excellent curability, preferred for use is at least one selected from the group consisting of 1,2-ethanediol di(meth)acrylate, 1,3-propanediol di(meth)acrylate, 1,4-butanediol di(meth)acrylate, 1,3-butanediol di(meth)acrylate, 1,5-pentanediol diacrylate, neopentyl glycol diacrylate, 1,6-hexanediol diacrylate, diethylene glycol di(meth)acrylate, glycerol di(meth)acrylate, and 1-(acryloyloxy)-3-(methacryloyloxy)-2-propanol. The content of polyfunctional polymerizable compound (e) having a Mw/n of less than 120 is preferably 0.001 to 5.0 parts by mass relative to total 100 parts by mass of the monofunctional polymerizable compound (a) and the polyfunctional polymerizable compound (b). A certain embodiment is, for example, an energy ray-curable coating material (A) for three-dimensional shaped articles that does not comprise the polyfunctional polymerizable compound (e) as a polymerizable compound.

An energy ray-curable coating material (A) for three-dimensional shaped articles of the present invention may comprise a polymerization accelerator (f) to improve photocurability, provided that it is not detrimental to the intent and purpose of the present invention. Examples of the polymerization accelerator (f) include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of imparting excellent curability to the coating material (A), preferred for use is at least one selected from the group consisting of ethyl 4-(N,N-dimethylamino)benzoate, n-butoxyethyl 4-(N,N-dimethylamino)benzoate, and 4-(N,N-dimethylamino)benzophenone.

An energy ray-curable coating material (A) for three-dimensional shaped articles of the present invention may

additionally comprise a filler (g) to adjust paste properties or to increase the mechanical strength of the cured product. Examples of the filler (g) include organic fillers, inorganic fillers, and organic-inorganic composite fillers. The filler (g) may be used alone, or two or more thereof may be used in combination.

Examples of materials of the organic fillers include polymethyl methacrylate, polyethyl methacrylate, a methyl methacrylate-ethyl methacrylate copolymer, crosslinked polymethyl methacrylate, crosslinked polyethyl methacrylate, polyester, polyamides, polycarbonates, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. The shape of the organic filler is not particularly limited, and the particle size of the filler may be appropriately selected for use.

Examples of materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. The shape of the inorganic filler is not particularly limited, and may be appropriately selected for use from, for example, irregularly shaped fillers and spherical fillers.

A polymer having no polymerizable group may be added to an energy ray-curable coating material (A) for three-dimensional shaped articles of the present invention, in order to alter properties such as flexibility and fluidity, provided that it is not detrimental to the intent and purpose of the present invention. Examples of such polymers include natural rubber, synthetic polyisoprene rubber, liquid polyisoprene rubber and hydrogenation products thereof, polybutadiene rubber, liquid polybutadiene rubber and hydrogenation products thereof, styrene-butadiene rubber, chloroprene rubber, ethylene-propylene rubber, acryl rubber, isoprene-isobutylene rubber, acrylonitrile-butadiene rubber, and styrene elastomers. Specific examples of other polymers that may be added include a polystyrene-polyisoprene-polystyrene block copolymer, a polystyrene-polybutadiene-polystyrene block copolymer, a poly($\alpha$-methylstyrene)-polybutadiene-poly($\alpha$-methylstyrene) block copolymer, a poly(p-methylstyrene)-polybutadiene-poly(p-methylstyrene) block copolymer, and hydrogenation products of these.

An energy ray-curable coating material (A) for three-dimensional shaped articles of the present invention may optionally comprise a softener. Examples of the softener include petroleum-base softeners such as paraffin, naphthene, and aromatic process oils, and vegetable oil-base softeners such as paraffin, peanut oil, and rosin. These softeners may be used alone, or two or more thereof may be used in combination. The softener content is not particularly limited, as long as it is not detrimental to the intent and purpose of the present invention. Typically, the softener content is at most 200 parts by mass, preferably at most 100 parts by mass relative to total 100 parts by mass of the monofunctional polymerizable compound (a) and the polyfunctional polymerizable compound (b).

An energy ray-curable coating material (A) for three-dimensional shaped articles of the present invention may comprise a known stabilizer to reduce degradation or adjust photocurability. Examples of the stabilizer include polymerization inhibitors, ultraviolet absorbers, antioxidants, cross-linking agents (for example, a metal ion releasing component, such as a polyvalent metal ion releasing filler).

Examples of the polymerization inhibitors include hydroquinone, hydroquinone monomethyl ether, dibutyl hydroquinone, dibutyl hydroquinone monomethyl ether, 4-t-butyl catechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to total 100 parts by mass of the monofunctional polymerizable compound (a) and the polyfunctional polymerizable compound (b).

An energy ray-curable coating material (A) for three-dimensional shaped articles of the present invention may comprise a known additive to adjust shades or paste properties. Examples of the additive include pigments, dyes, and thickeners.

Another embodiment is, for example, an energy ray-curable material kit for three-dimensional shaping comprising an energy ray-curable coating material (A) for three-dimensional shaped articles, and an energy ray-curable composition (B) for three-dimensional shaping.

Energy Ray-Curable Composition (B) for Three-Dimensional Shaping

An energy ray-curable material kit for three-dimensional shaping of the present invention is a kit configured from an energy ray-curable coating material (A) for three-dimensional shaped articles, and an energy ray-curable composition (B) for three-dimensional shaping,

the energy ray-curable coating material (A) for three-dimensional shaped articles comprising the monofunctional polymerizable compound (a) and/or the polyfunctional polymerizable compound (b), and the polymerization initiator

(c), wherein the polyfunctional polymerizable compound (b) has a Mw/n of 120 or more, where Mw is a molecular weight of the polyfunctional polymerizable compound (b), and n is the number of polymerizable groups per molecule, the energy ray-curable composition (B) for three-dimensional shaping comprising a polymerizable compound and a polymerization initiator.

In view of increasing affinity for the energy ray-curable coating material (A) for three-dimensional shaped articles, the polymerizable compound contained in the energy ray-curable composition (B) for three-dimensional shaping preferably comprises at least one selected from the monofunctional polymerizable compound (a) and the polyfunctional polymerizable compound (b). In view of excellence of fabricability by stereolithography, the energy ray-curable composition (B) for three-dimensional shaping in certain embodiments may be one containing no monofunctional polymerizable compound (a1) as a polymerizable compound, or one containing no monofunctional polymerizable compound (a2) as a polymerizable compound, or may be one containing neither the monofunctional polymerizable compound (a1) nor the monofunctional polymerizable compound (a2) as a polymerizable compound.

The polymerization initiator contained in the energy ray-curable composition (B) for three-dimensional shaping is preferably the photopolymerization initiator (c1), more preferably at least one selected from the group consisting of a (bis)acylphosphine oxide and a salt thereof, an oxime ester compound, and an acridine compound. Particularly preferred are (bis)acylphosphine oxides and salts thereof. It is also preferable to use at least one selected from the group consisting of a (bis)acylphosphine oxide and an $\alpha$-diketone because the energy ray-curable composition for three-dimensional shaping can have excellent photocurability in the ultraviolet region and visible light region, and shows sufficient photocurability regardless of whether the light source used is a laser, a halogen lamp, a light emitting diode (LED), or a xenon lamp.

The mechanical characteristics and optical characteristics of a shaped article obtained from the energy ray-curable composition (B) for three-dimensional shaping are not particularly limited. However, in order for a coating material (A) of the present invention to exhibit the surface smoothing effect without impairing the properties of the shaped article, the mechanical characteristics and optical characteristics are preferably similar to the mechanical characteristics and optical characteristics of the energy ray-curable coating material (A) for three-dimensional shaped articles. Specifically, it is preferable that the difference between the tensile elastic modulus of a cured product of the energy ray-curable coating material (A) for three-dimensional shaped articles and the tensile elastic modulus of a shaped article formed of the energy ray-curable composition (B) for three-dimensional shaping be 200 MPa or less, or that the tensile elastic modulus of a cured product of the energy ray-curable coating material (A) for three-dimensional shaped articles be smaller than the tensile elastic modulus of a shaped article formed of the energy ray-curable composition (B) for three-dimensional shaping. The tensile elastic modulus difference is more preferably 150 MPa or less, even more preferably 100 MPa or less. It is also preferable that the difference between the transparency $\Delta L_A$ of a cured product of the energy ray-curable coating material (A) for three-dimensional shaped articles and the transparency $\Delta L_B$ of a shaped article formed of the energy ray-curable composition (B) for three-dimensional shaping be 20 or less. The transparency difference is more preferably 15 or less, even more preferably 10 or less.

An energy ray-curable coating material (A) for three-dimensional shaped articles of the present invention has excellent toughness. This enables a coating material (A) of the present invention to smooth the surface of a shaped article requiring flexibility and high toughness, and impart functionality to the shaped article without imparting the properties of the article. As an example, such a shaped article is most suited for dental treatment, including, for example, an orthodontic mouthpiece, a mouthpiece for treating sleep disorders, a mouthpiece for treating temporomandibular joint disorders, a mouthpiece for protecting teeth or temporomandibular joint, and a dental material such as a denture base material; and intraoral use, including a mouthguard as sporting protective gear against external forces. The shape of a cured product using a coating material (A) of the present invention can be varied depending on use, along with the cured product of the energy ray-curable composition (B) for three-dimensional shaping. In a coating material (A) of the present invention, the type and content of the components (monofunctional polymerizable compound (a) and/or polyfunctional polymerizable compound (b), polymerization initiator (c), and optional components (such as solvent (d), polymerization accelerator (f), filler (g), a polymer, a softener, a stabilizer, and an additive)) can be optionally adjusted for different uses.

In cured products of the energy ray-curable composition for three-dimensional shaping, a flexible cured product has a looser molecular-chain network than a hard cured product, and the water resistance, antifouling properties, and antimicrobial properties of the cured product may decrease because such a loose molecular-chain network is more susceptible to entry of water and bacteria. However, because polymerizable compounds containing an alicyclic hydrocarbon group, a chain alkyl group having 8 or more carbon atoms, a fluoro group, and a silyl group, or polymerizable compounds having a large Mw/n value have low curability from among the polymerizable compounds that excel in water resistance, antifouling properties, and antimicrobial properties, these polymerizable compounds impair fabricability when contained in large amounts in the energy ray-curable composition (B) for three-dimensional shaping. Even if fabricable, such polymerizable compounds cause roughness and tackiness, and the surface properties decrease as a result of impaired curability, particularly at the surface affected by oxygen inhibition. These polymerizable compounds also have

poor compatibility to other components, and the transparency often decreases as a result of phase separation. Polymerizable compounds that excel in properties such as water resistance, antifouling properties, and antimicrobial properties are also generally expensive. In polymerizable compounds that excel in water resistance, antifouling properties, and antimicrobial properties, a polymerizable compound having a small molecular weight and a boiling point at normal pressure of 250°C or less easily vaporizes. This can be harmful to the worker because such a polymerizable compound, when contained in large amounts in the energy ray-curable composition (B) for three-dimensional shaping, causes changes in the composition over time, or produces an odor as it vaporizes during three-dimensional shaping. In polymerizable compounds that excel in water resistance, antifouling properties, and antimicrobial properties, a polymerizable compound containing an aromatic ring absorbs visible light, and tends to impart a yellow color or discolor the shaped article when contained in large amounts in the energy ray-curable composition (B) for three-dimensional shaping. Accordingly, it is particularly effective when these polymerizable compounds are contained in an energy ray-curable coating material (A) for three-dimensional shaped articles of the present invention because it allows the polymerizable compounds to efficiently provide high functionality to article's surface without impairing the fabricability of the energy ray-curable composition (B) for three-dimensional shaping, the stability of the composition, or the appearance of the shaped article, using reduced amounts of expensive polymerizable compounds.

The characteristics of an energy ray-curable coating material (A) for three-dimensional shaped articles of the present invention capable of smoothing the surface of a flexible shaped article or a high-toughness shaped article or imparting functionality to such shaped articles without impairing article's properties can be exploited in a wide range of applications. For example, an energy ray-curable coating material (A) for three-dimensional shaped articles of the present invention can be used to smooth the surface or add high functionality to a flexible shaped article used in industry. An energy ray-curable coating material (A) for three-dimensional shaped articles of the present invention can smooth the surface of an elastic material, or impart water resistance or antimicrobial properties to the surface of a polyurethane-like shaped article used for shoe soles.

Another embodiment of the present invention is, for example, a method that comprises producing a three-dimensional shaped article by stereolithography using the energy ray-curable composition (B) for three-dimensional shaping, coating the three-dimensional shaped article with the energy ray-curable coating material (A) for three-dimensional shaped articles, and integrating the energy ray-curable coating material (A) for three-dimensional shaped articles and the cured product of the energy ray-curable composition (B) for three-dimensional shaping by photoirradiation and/or heat treatment.

For stereolithography using the energy ray-curable composition (B) for three-dimensional shaping of the present invention, a conventionally known stereolithography method and device may be used (for example, a stereolithography device such as the DigitalWax® 028J-Plus manufactured by DWS). In the present invention, the light energy used to cure the resin is preferably an active energy beam. As used herein, "active energy beam" means an energy ray capable of curing a photocurable resin composition, and includes, for example, ultraviolet light, an electron beam, X-rays, radiant rays, and high-frequency waves. For example, the active energy beam may be ultraviolet light of 300 to 400 nm wavelengths. The light source of active energy beam may be, for example, a laser such as an Ar laser or a He-Cd laser; or a lighting such as a halogen lamp, a xenon lamp, a metal halide lamp, an LED, a mercury lamp, and a fluorescent lamp. Lasers are particularly preferred. When the light source is a laser, the fabrication time can be reduced by increasing the energy level, and a high-precision three-dimensional shaped article can be obtained by taking advantage of the desirable convergence of a laser beam.

For stereolithography using the energy ray-curable composition (B) for three-dimensional shaping of the present invention, a conventionally known method and a conventionally known stereolithography system may be used, and the method and device are not particularly limited, as noted above. A typical example of a stereolithography method preferred for use in the present invention is a method that produces a desired final three-dimensional shaped article through a repeated procedure that includes a step of forming a cured layer by selectively applying an active energy beam to the energy ray-curable composition (B) for three-dimensional shaping so as to obtain a cured layer having a desired pattern, and a step of continuously forming another cured layer on the previously formed cured layer by similarly applying an active energy beam to a newly supplied, uncured liquid of energy ray-curable composition (B) for three-dimensional shaping.

A three-dimensional shaped article obtained by three-dimensional shaping is not limited to a particular structure, shape, or size, and these may be decided according to use. Typical examples of areas to which the stereolithography method of the present invention is applicable include production of various models and molds, including, for example, models for assessing external designs in a designing process; models for checking functions of components and parts; resin molds for making molds; base models for making dies; and direct molds for prototype dies. More specifically, the stereolithography method of the present invention is applicable to, for example, production of models or work models for precision components and parts, electrical and electronic components, furniture, architectural structures, automobile parts, various containers and vessels, castings, dies, and matrices. By taking advantage of the excellent flexibility and elastic recovery of the energy ray-curable composition for three-dimensional shaping, the stereolithography method of

the present invention can be very effectively used for, for example, the midsole and outsole of a shoe, joint supports for athletes and disabled persons, and training organ models for surgical procedures.

The three-dimensional shaped article obtained may be post-cured with applied light or heat after being coated with the energy ray-curable coating material (A) for three-dimensional shaped articles, or may be coated with the energy ray-curable coating material (A) for three-dimensional shaped articles after improving mechanical characteristics, shape stability, or other properties by, for example, post-curing the article under applied light or heat. In view of providing the effect to reduce detachment of the coating layer formed as a cured product of energy ray-curable coating material (A) for three-dimensional shaped articles, it is preferable to form a covalent bond between the polymerizable compound in the energy ray-curable coating material (A) for three-dimensional shaped articles and the polymerizable compound in the energy ray-curable composition (B) for three-dimensional shaping. To this end, it is preferable that post-cure be performed after coating the energy ray-curable composition (B) for three-dimensional shaping with the energy ray-curable coating material (A) for three-dimensional shaped articles.

A conventionally known method may be used for coating of the three-dimensional shaped article of the energy ray-curable composition (B) for three-dimensional shaping with the energy ray-curable coating material (A) for three-dimensional shaped articles. Examples of such methods include application with a writing brush, a brush, or a roller; spraying with a sprayer; and dipping in the energy ray-curable coating material (A) for three-dimensional shaped articles. In the case where washing with an organic solvent is incorporated to remove uncured polymerizable compounds from the three-dimensional shaped article of the energy ray-curable composition (B) for three-dimensional shaping, the energy ray-curable coating material (A) for three-dimensional shaped articles may be contained in the organic solvent. Preferably, coating is performed by dipping because it enables formation of a large number of strong covalent bonds by allowing the energy ray-curable coating material (A) for three-dimensional shaped articles to thoroughly penetrate the three-dimensional shaped article of the energy ray-curable composition (B) for three-dimensional shaping. The polymerizable compound in the energy ray-curable coating material (A) for three-dimensional shaped articles constituting the coating layer, and the polymerizable compound in the energy ray-curable composition (B) for three-dimensional shaping constituting the three-dimensional shaped article can be determined as having formed a covalent bond when there is no detachment of the coating layer upon immersion in organic solvent.

The energy ray-curable coating material (A) for three-dimensional shaped articles may cover the whole or part of the three-dimensional shaped article of the energy ray-curable composition (B) for three-dimensional shaping. For example, only the desired part of the three-dimensional shaped article may be coated by masking the three-dimensional shaped article. However, in view of reducing detachment of the coating layer formed as a cured product of the energy ray-curable coating material (A) for three-dimensional shaped articles, it is preferable to cover the whole three-dimensional shaped article of the energy ray-curable composition (B) for three-dimensional shaping.

The energy ray-curable coating material (A) for three-dimensional shaped articles may be coated once or multiple times. When coating the energy ray-curable coating material (A) for three-dimensional shaped articles more than once, a coating material (A) of the same composition may be coated in a repeated fashion, or the three-dimensional shaped article may be coated first with a coating material (A) of a certain composition, and then with a coating material (A) of a different composition after integrating the first coating material (A) with the three-dimensional shaped article by photoirradiation and/or heat treatment.

The thickness of the coating layer formed of the energy ray-curable coating material (A) for three-dimensional shaped articles is not particularly limited. In view of precision, the coating layer thickness is preferably 250 $\mu$m or less, more preferably 100 $\mu$m or less, even more preferably 50 $\mu$m or less. The coating layer thickness can be measured with, for example, a spectroscopic ellipsometer (Horiba Ltd.) by spectroscopic ellipsometry.

EXAMPLES

The following describes the present invention in greater detail by way of Examples. However, the present invention is in no way limited by the following Examples, and various changes may be made by a person with ordinary skill in the art within the technical idea of the present invention.

The components used for the energy ray-curable coating material (A) for three-dimensional shaped articles and the energy ray-curable composition (B) for three-dimensional shaping according to Examples and Comparative Examples are presented below, along with the abbreviations.

Monofunctional Polymerizable Compound (a)

Monofunctional Polymerizable Compound (a1)

ACMO: N-acryloylmorpholine (manufactured by KJ Chemicals Corporation; polymer Tg: 145°C)
MMA: Methyl methacrylate (manufactured by Fujifilm Wako Pure Chemical Industries, Ltd.; Tg: 105°C)

Monofunctional Polymerizable Compound (a2)

POB-A: m-Phenoxybenzyl acrylate (manufactured by Kyoeisha Chemical Co., Ltd.; polymer Tg: 0°C or less)
EPPA: Ethoxylated-o-phenylphenol acrylate (manufactured by Shin-Nakamura Chemical Co., Ltd.; polymer Tg: 35°C)
8FMA: 1H,1H,5H-Octafluoropentyl methacrylate (manufactured by Tokyo Chemical Industry Co., Ltd.; polymer Tg: 20°C or less)
SPE: N-Methacryloyloxyethyl-N,N-dimethylammonium-α-N-propyl sulfobetaine (manufactured by Tokyo Chemical Industry Co., Ltd.; polymer Tg: 20°C or less)
MPC: 2-Methacryloyloxyethyl phosphorylcholine (manufactured by Tokyo Chemical Industry Co., Ltd.; polymer Tg: 20°C or less)

Polyfunctional Polymerizable Compound (b)

Polyfunctional Polymerizable Compound (b1-1)

UDMA: 2,2,4-Trimethylhexamethylene bis(2-carbamoyloxyethyl)dimethacrylate (manufactured by Kyoeisha Chemical Co., Ltd.; Mw = 471, n = 2, Mw/n = 236, polymer Tg: 100°C)
TCDDMA: Tricyclodecane dimethanol diacrylate (manufactured by Shin-Nakamura Chemical Co., Ltd.; Mw = 304, n = 2, Mw/n = 152, polymer Tg: 200°C or more)
TM-100: Silsesquioxane monomer (MAC-SQ TM-100 manufactured by Toagosei Co., Ltd.; Mw/n = 179 (double bond equivalent; a value disclosed by the maker), polymer Tg: 100°C)

Polyfunctional Polymerizable Compound (b2)

EBECRYL 8465: Aliphatic urethane acrylate (manufactured by Daicel allnex; Mw = 1,500 (a value disclosed by the maker), n = 3, polymer Tg: 36°C)
EBECRYL 8807: Aliphatic urethane acrylate (manufactured by Daicel allnex; Mw = 1,000 (a value disclosed by the maker), n = 2, polymer Tg: 32°C)
UF-C01: Polyether urethane acrylate (manufactured by Kyoeisha Chemical Co., Ltd.; Mw = 10,000, n = 2, Mw/n = 5,000, polymer Tg: 41°C)
8SS-723: Silicone acryl polymer (ACRIT 8SS-723 manufactured by Taisei Fine Chemical Co,. Ltd., Mw/n = 338 (double bond equivalent; a value disclosed by the maker), polymer Tg: 20°C or less)
8WX-018: Acryl polymer (ACRIT 8WX-018 manufactured by Taisei Fine Chemical Co,. Ltd., containing a quaternary ammonium salt group on side chain, Mw/n = 700 (double bond equivalent; a value disclosed by the maker), polymer Tg: 20°C or less)
3G: Triethylene glycol dimethacrylate (Tg: 0°C)

Polyfunctional Polymerizable Compound (e)

DGB: 1,4-Diglycidyloxybutane (manufactured by Tokyo Chemical Industry Co., Ltd.; Mw = 202, n = , Mw/n = 101, polymer Tg: 200°C or more)
IATE: Triglycidyl isocyanurate (manufactured by Tokyo Chemical Industry Co., Ltd., Mw = 297, n = 3, Mw/n = 99, polymer Tg: 200°C or more)
MAA: N,N-Methylenebisacrylamide (manufactured by Tokyo Chemical Industry Co., Ltd., Mw = 154, n = 2, Mw/n = 77, polymer Tg: 200°C or more)
DPE-6A: Dipentaerythritol hexaacrylate (manufactured by Kyoeisha Chemical Co., Ltd.; Mw = 578, n = 6, Mw/n = 96)

A disc-shaped cured product, measuring 25 mm in diameter and 1.0 mm in thickness, was prepared from each of the monofunctional polymerizable compound (a), the polyfunctional polymerizable compound (b), and the polyfunctional polymerizable compound (e), using a photoirradiator (Otofolash® G171, manufactured by Envision TEC), and the peak temperature of tanΔ of each cured product was measured as a polymer glass transition temperature, using a dynamic viscoelasticity meter (a rotary rheometer AR 2000 manufactured by TA instrument).

Polymerization Initiator (c)

TPO: 2,4,6-Trimethylbenzoyldiphenylphosphine oxide (manufactured by IGM Resins)
Omnirad 784: Bis($\eta^5$-2,4-cyclopentadien-1-yl)-bis(2,6-difluoro-3-(1H-pyrrol-1-yl)-phenyl)titanium (manufactured by IGM Resins)
BPO: Benzoyl peroxide (manufactured by NOF Corporation)
UVI-6970 (Cationic photopolymerization initiator, triarylsulfonium salt, manufactured by Union Carbide Corporation)

Polymerization Inhibitor

BHT: 3,5-Di-t-butyl-4-hydroxytoluene (manufactured by Fujifilm Wako Pure Chemical Industries, Ltd.)

Solvent

Ethanol (manufactured by Fujifilm Wako Pure Chemical Industries, Ltd.)

Examples 1 to 25 and Comparative Examples 1 to 4

The following experiment was conducted by simulating an energy ray-curable material kit for three-dimensional shaping including an energy ray-curable coating material (A) for three-dimensional shaped articles and an energy ray-curable composition (B) for three-dimensional shaping, as follows.
First, the components shown in Table 1 were mixed at ordinary temperature (20°C ± 15°C, JIS (Japanese Industrial Standards) Z 8703:1983) in the amounts shown in the table to prepare pastes as energy ray-curable compositions (B) for three-dimensional shaping according to Examples 1 to 25 and Comparative Examples 1 to 4.
Separately, the components shown in Tables 2, 3, 4, and 5 were mixed at ordinary temperature (20°C ± 15°C, JIS (Japanese Industrial Standards) Z 8703:1983) in the amounts shown the tables to prepare pastes as energy ray-curable coating materials (A) for three-dimensional shaped articles according to Examples 1 to 25 and Comparative Examples 1 to 4.
From the paste of energy ray-curable composition (B) for three-dimensional shaping obtained in each Example and Comparative Example, a sheet measuring 1 mm in thickness, 8 cm in length, and 2 cm in width was formed with a stereolithography device (DigitalWax® 028J-Plus, manufactured by DWS) under 0.1 mm slicing conditions. The excess polymerizable compounds were removed by air blowing and washing with ethanol. The three-dimensional shaped article obtained was immersed in a bath containing the energy ray-curable coating material (A) for three-dimensional shaped articles according to each Example and Comparative Example. After being left to stand in the bath for 5 minutes, the sheet was taken out of the bath, and the excess polymerizable compounds were removed by blowing air. For tests using dumbbell specimens, the sheet was punched into a specimen of a desired shape with a punching blade at this stage. The energy ray-curable composition for three-dimensional shaping, and the energy ray-curable coating material (A) for three-dimensional shaped articles were then polymerized with a photoirradiator (Otoflash® G171, manufactured by Envision TEC) and/or a dental laboratory polymerization apparatus (thermal polymerizer KL-400, manufactured by SK Medical Electronics Co., Ltd.) to obtain a three-dimensional shaped article having a coating layer, specifically, a sheet formed of the three-dimensional shaped article as a cured product of the energy ray-curable composition (B) for three-dimensional shaping, and the coating layer as a cured product of the energy ray-curable coating material (A) for three-dimensional shaped articles.
Separately, the paste of energy ray-curable coating material (A) for three-dimensional shaped articles prepared for each Example and Comparative Example was filled into a sheet-shaped metal frame measuring 1 mm in thickness, 8 cm in length, and 2 cm in width, and polymerized with a photoirradiator (Otoflash® G171, manufactured by Envision TEC) and/or a thermal polymerizer KL-400 (manufactured by SK Medical Electronics Co., Ltd.) to obtain a specimen as a cured product of only the energy ray-curable coating material (A) for three-dimensional shaped articles. These specimens were used for the evaluations of coating material properties ("Properties of coating material" in Tables 2 to 5).

Surface Smoothness

As a pretreatment for smoothness measurement, a metal coating, about 50 nm thick, was formed by sputtering on the surface of a three-dimensional shaped article having the coating layer. The surface roughness Ra (arithmetic mean roughness Ra) was then measured with a color 3D laser microscope (VK-9700, manufactured by Keyence) (n = 5). The mean value of measured values was evaluated using the following criteria.

++: Surface roughness Ra is 0.05 mm or less
+: Surface roughness Ra is more than 0.05 mm and 0.08 mm or less
-: Surface roughness Ra is more than 0.08 mm

Tensile Properties

For cured products of the energy ray-curable compositions (B) for three-dimensional shaping and the energy ray-curable coating materials (A) for three-dimensional shaped articles according to Examples and Comparative Examples, a specimen having the same dimensions as the dumbbell-8 specimen described in JIS K 6251:2010 (Rubber, vulcanized or thermoplastics-Determination of tensile stress-strain properties) was prepared from a sheet coated with a cured product of the energy ray-curable coating material (A) for three-dimensional shaped articles, using a punching blade. For each specimen, a tensile test was conducted at a test speed of 500 mm/min (n = 5). The mean value of measured values was presented as "Properties after coating" in Tables 2 to 5. The cured product of the energy ray-curable coating material (A) for three-dimensional shaped articles can be said as being not detrimental to the original quality of the three-dimensional shaped article when the value of tensile elongation in the test is equal to or greater than the value of the three-dimensional shaped article solely formed of the energy ray-curable composition (B) for three-dimensional shaping. Similarly, the cured product of the energy ray-curable coating material (A) for three-dimensional shaped articles can be said as being not detrimental to the original quality of the three-dimensional shaped article when the difference between the tensile elastic modulus of the cured product of the energy ray-curable coating material (A) for three-dimensional shaped articles and the tensile elastic modulus of the three-dimensional shaped article of the energy ray-curable composition (B) for three-dimensional shaping is 200 MPa or less, or when the tensile elastic modulus of the cured product of the energy ray-curable coating material (A) for three-dimensional shaped articles is smaller than the tensile elastic modulus of the three-dimensional shaped article of the energy ray-curable composition (B) for three-dimensional shaping in the test.

Toughness

In the tensile test, the coating layer of the energy ray-curable coating material (A) for three-dimensional shaped articles was visually evaluated for the presence or absence of breakage at fracture (n = 5). The cured product of the energy ray-curable coating material for three-dimensional shaped articles can be said as having high toughness when there is no cracking. The following criteria were used.

++: No cracking
+: No breakage, but cracking occurred in one or more of the specimens
-: Breakage occurred in one or more of the specimens

Water Resistance

The cured product of the energy ray-curable composition (B) for three-dimensional shaping according to each Example and Comparative Example was immersed in 37°C water for 24 hours, and was measured for tensile elastic modulus by conducting a tensile test in the manner described above (n = 5). From the measured values, the percentage retention of tensile elastic modulus was calculated using the formula below. The mean values are presented in Table 3. The cured product can be said as having excellent water resistance when at least 70% of the initial tensile elastic modulus is retained after 24-hour immersion in 37°C water.

$$\text{Percentage retention of tensile elastic modulus (\%)} = [\text{tensile elastic modulus after immersion in water (MPa)} / \text{initial tensile elastic modulus (MPa)}] \times 100$$

Antimicrobial Properties

The cured product of the energy ray-curable composition (B) for three-dimensional shaping according to each Example and Comparative Example was subjected to an antimicrobial test, according to JIS Z 2801:2000. A bacterial solution containing Escherichia coli, and a bacterial solution containing Staphylococcus aureus were dropped in each cured product, and the number of viable bacteria after 24 hours was counted (n = 3). Under the same conditions, Escherichia coli was grown on a cured product of energy ray-curable composition (B) for three-dimensional shaping with no coating (standard sample), and the number of viable bacteria was counted (n = 3). The common logarithm of X/Y was determined as the antimicrobial activity value, where X is the number of viable bacteria in standard sample, and Y is the number of viable bacteria in a sample of Example. The antimicrobial properties were evaluated using the following criteria.

+: Antimicrobial activity value is 2.0 or higher
-: Antimicrobial activity value is less than 2.0

Stain Resistance

The cured product of the energy ray-curable composition (B) for three-dimensional shaping according to each Example and Comparative Example was subjected to a stain resistance test. Before testing, the cured products were measured for *b values of the chromaticity (color space) of the L*a*b* color system (JIS Z 8781-4:2013), using a spectrophotometer (SE 2000, D65 illuminant; manufactured by Nippon Denshoku Industries Co., Ltd.). The cured products were immersed in a pigment solution prepared with turmeric/water = 2/100 (w/w), and left to stand at 37°C for 24 hours (n = 3). After 24 hours, the surface was rinsed with distilled water, and, after wiping off water, the *b value was measured in the same manner. The difference ($\Delta$b) in *b value before and after immersion was determined using the following formula.

$$\Delta b = {}^*b \text{ (after immersion)} - {}^*b \text{ (before immersion)}$$

Water resistance is excellent when there is little staining with the yellow color of turmeric, that is, a small $\Delta$b value. The stain resistance was evaluated as "+" when the *b value was 15 or less, and "++" when the *b value was 10 or less.

Transparency

A disc-shaped shaped article measuring 15.0 mm in diameter and 1.0 mm in thickness was produced for the composition of each Example and Comparative Example shown in Tables 1 and 2, using a stereolithography device (DigitalWax® 028J-Plus, manufactured by DWS). After washing the shaped article with methanol, the unpolymerized monomer compounds were removed, and a cured product was obtained after further polymerization conducted for 90 seconds with a dental laboratory LED polymerization apparatus (Alpha-Light V manufactured by J. MORITA TOKYO MFG. Corp. under this trade name). The cured products were then measured for *L values of the chromaticity (color space) of the L*a*b* color system (JIS Z 8781-4:2013), using a spectrophotometer (SE 2000, D65 illuminant; manufactured by Nippon Denshoku Industries Co., Ltd.) (n = 5), and transparency $\Delta$L was measured. Transparency $\Delta$L is defined by the formula below. The mean values calculated from measured values are presented as values of transparency $\Delta$L in Tables 1 to 5.

$$\Delta L = L^*W - L^*B,$$

where L*W represents the lightness index L* in the L*a*b* color system of JIS Z 8781-4:2013 measured against a white background, and L*B represents the lightness index L* of the L*a*b*color system measured against a black background.

[Table 1]

| | | (B)-1 | (B)-2 |
|---|---|---|---|
| Composition of energy ray-curable composition for three-dimensional shaping (parts by mass) | DPE-6A | 60 | |
| | EBECRYL 8807 | | 60 |
| | 3G | 40 | 40 |
| | TPO | 1.0 | 1.0 |
| | BHT | 0.05 | 0.05 |
| Properties | Tensile elongation [%] | 0.25 | 31 |
| | Tensile elastic modulus [MPa] | 780 | 520 |
| | ΔL | 91 | 95 |

[Table 2]

| | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Com. Ex. 1 | Com. Ex. 2 | Com. Ex. 3 | Com. Ex. 4 |
|---|---|---|---|---|---|---|---|---|---|---|---|---|---|---|---|---|
| Composition of coating material (parts by mass) | ACMO (a1) | 50 | | | | | | | | | | | | | | |
| | MMA(a1) | | 50 | | 50 | | 80 | | | | | | | | | |
| | POB-A (a2) | 50 | 50 | 100 | | | 10 | 70 | | | | | | | | |
| | EPPA (a2) | | | | 40 | | | | | | | | | | | |
| | UDMA (b1-1) | | | | | 50 | 10 | | | | | | | | | |
| | TCDDMA (b1-1) | | | | | | | 30 | | | | | | | | |
| | EBECRYL8465 (b2) | | | | | | | | 60 | | 60 | | | | | |
| | UF-C01 (b2) | | | | 10 | | | | | 30 | | 30 | | | | |
| | DGB | | | | | | | | | | | | | 100 | | |
| | IATE | | | | | | | | | | | | | | 100 | |
| | MAA | | | | | | | | | | | | | | | 100 |
| | TPO | 1.0 | 1.0 | 1.0 | | 1.0 | 1.0 | | 1.0 | 1.0 | 1.0 | 1.0 | | | | 1.0 |
| | Omnirad784 | | | | 1.0 | | | | | | | | | | | |
| | BPO | | | | | | 0.1 | 1.0 | | | | | | | | |
| | UVI-6970 | | | | | | | | | | | | | 1.0 | 1.0 | |
| | BHT | 0.05 | 0.05 | 0.05 | 0.05 | 0.05 | 0.05 | 0.05 | 0.05 | 0.05 | 0.05 | 0.05 | | 0.05 | 0.05 | 0.05 |
| | Ethanol | | | | | 50 | | | | 20 | | 20 | | | | |
| | Composition of shaped article | (B)-2 | (B)-2 | (B)-2 | (B)-2 | (B)-2 | (B)-2 | (B)-2 | (B)-2 | (B)-2 | (B)-1 | (B)-1 | (B)-2 | (B)-1 | (B)-2 | (B)-2 |
| Properties of coating material | Tensile elastic modulus [MPa] | 420 | 400 | N.D. | 660 | 710 | 730 | 640 | 530 | 550 | 530 | 550 | N/A | 860 | 820 | 740 |
| | Difference of elastic modulus from (B) | 100 | 120 | 520 | 140 | 190 | 210 | 120 | 10 | 30 | 250 | 230 | N/A | -340 | -300 | -220 |
| | ΔL | 95 | 93 | 94 | 94 | 91 | 94 | 94 | 93 | 94 | 93 | 94 | N/A | 90 | 90 | 88 |
| | Difference of ΔL from (B) | 0 | 2 | 1 | 1 | 4 | 1 | 1 | 2 | 1 | 2 | 3 | N/A | 5 | 5 | 7 |

(continued)

| | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Com. Ex. 1 | Com. Ex. 2 | Com. Ex. 3 | Com. Ex. 4 |
|---|---|---|---|---|---|---|---|---|---|---|---|---|---|---|---|---|
| Properties after coating | Surface smoothness | ++ | ++ | ++ | ++ | ++ | ++ | ++ | ++ | ++ | ++ | ++ | N/A | ++ | ++ | ++ |
| | Tensile elongation [%] | 32 | 30 | 40 | 34 | 30 | 32 | 31 | 32 | 32 | 0.5 | 0.7 | 31 | 0.21 | 15 | 10 |
| | Tensile elastic modulus [MPa] | 500 | 560 | 480 | 520 | 520 | 540 | 520 | 520 | 520 | 520.0 | 530 | 530 | 810 | 800 | 780 |
| | Toughness | ++ | ++ | ++ | ++ | ++ | + | ++ | ++ | ++ | ++ | ++ | N/A | - | - | - |

In the table, N.D. means that tensile elastic modulus was unmeasurable because of softness of cured product. N/A means unmeasurable.

[Table 3]

| | | Ex. 12 | Ex. 13 | Ex. 14 | Ex. 15 | Ex. 16 | Ex. 17 | Com. Ex. 1 |
|---|---|---|---|---|---|---|---|---|
| Composition of coating material (parts by mass) | MMA(a1) | 100 | 80 | | | | | No coating |
| | POB-A (a2) | | | 100 | | 70 | | |
| | UDMA (b1-1) | | 20 | | 50 | | 40 | |
| | TCDDMA (b1-1) | | | | | 30 | | |
| | 8SS-723 (b2) | | | | | | 30 | |
| | TPO | 1.0 | 1.0 | 1.0 | 1.0 | 1.0 | 1.0 | |
| | BHT | 0.05 | 0.05 | 0.05 | 0.05 | 0.05 | 0.05 | |
| | Ethanol | | | | 50 | | 30 | |
| | Composition of shaped article | (B)-2 | (B)-2 | (B)-2 | (B)-2 | (B)-2 | (B)-2 | (B)-2 |
| Properties of coating material | Tensile elastic modulus [MPa] | 700 | 640 | N.D. | 710 | 340 | 380 | N/A |
| | Difference of elastic modulus from (B) | 180 | 120 | 520 | 190 | 180 | 140 | N/A |
| | ΔL | 92 | 90 | 94 | 91 | 94 | 85 | N/A |
| | Difference of ΔL from (B) | 3 | 5 | 1 | 4 | 1 | 10 | N/A |
| Properties after coating | Surface smoothness | ++ | ++ | ++ | ++ | ++ | ++ | N/A |
| | Tensile elongation [%] | 26 | 28 | 31 | 30 | 31 | 30 | 31 |
| | Toughness | ++ | ++ | ++ | ++ | ++ | ++ | N/A |
| | Water resistance Percentage retention of tensile elastic modulus [%] | 89 | 85 | 74 | 71 | 74 | 71 | 62 |
| In the table, N.D. means that tensile elastic modulus was unmeasurable because of softness of cured product. N/A means unmeasurable. | | | | | | | | |

[Table 4]

| | | Ex.18 | Ex.19 | Ex. 20 | Ex. 21 | Com. Ex. 1 |
|---|---|---|---|---|---|---|
| Composition of coating material (parts by mass) | 8FMA (a2) | 100 | | | | No coating |
| | SPE (a2) | | 100 | | | |
| | MPC (a2) | | | 80 | | |
| | UDMA (b1-1) | | | 20 | 20 | |
| | 8WX-018 (b2) | | | | 60 | |
| | TPO | 1.0 | 1.0 | 1.0 | 1.0 | |
| | BHT | 0.05 | 0.05 | 0.05 | 0.05 | |
| | Ethanol | | | 20 | 20 | |
| | Composition of shaped article | (B)-2 | (B)-2 | (B)-2 | (B)-2 | (B)-2 |

(continued)

| | | | Ex.18 | Ex.19 | Ex. 20 | Ex. 21 | Com. Ex. 1 |
|---|---|---|---|---|---|---|---|
| Properties of coating material | Tensile elastic modulus [MPa] | | 290 | 330 | 410 | 320 | N/A |
| | Difference of elastic modulus from (B) | | 230 | 140 | 140 | 200 | N/A |
| | ΔL | | 84 | 90 | 92 | 92 | N/A |
| | Difference of ΔL from (B) | | 11 | 5 | 3 | 3 | N/A |
| Properties after coating | Surface smoothness | | ++ | ++ | ++ | ++ | N/A |
| | Tensile elongation [%] | | 34 | 29 | 32 | 32 | 31 |
| | Toughness | | ++ | ++ | ++ | ++ | N/A |
| | Antimicrobial properties | Viable bacteria count | $1.2 \times 10^3$ | $2.0 \times 10^2$ | $3.0 \times 10^1$ | $1.8 \times 10^2$ | $3.6 \times 10^6$ |
| | | In(X/Y) | 3.5 | 4.3 | 5.1 | 3.3 | 0 |
| | | | + | + | + | + | |
| In the table, N/A means unmeasurable. | | | | | | | |

[Table 5]

| | | Ex. 22 | Ex. 23 | Ex. 24 | Ex. 25 | Com. Ex. 1 |
|---|---|---|---|---|---|---|
| Composition of coating material (parts by mass) | 8FMA (a2) | 80 | 60 | 60 | | No coating |
| | MPC (a2) | | | | 80 | |
| | UDMA (b1-1) | 20 | 40 | 20 | 20 | |
| | TM-100 (b2) | | | 20 | | |
| | TPO | 1.0 | 1.0 | 1.0 | 1.0 | |
| | BHT | 0.05 | 0.05 | 0.05 | 0.05 | |
| | Ethanol | | | 20 | 20 | |
| | Composition of shaped article | (B)-2 | (B)-2 | (B)-2 | (B)-2 | (B)-2 |
| Properties of coating material | Tensile elastic modulus [MPa] | 350 | 390 | 330 | 410 | N/A |
| | Difference of elastic modulus from (B) | 170 | 130 | 190 | 140 | N/A |
| | ΔL | 90 | 92 | 87 | 92 | N/A |
| | Difference of ΔL from (B) | 5 | 3 | 8 | 3 | N/A |
| Properties after coating | Surface smoothness | ++ | ++ | ++ | ++ | N/A |
| | Tensile elongation [%] | 30 | 25 | 29 | 32 | 31 |
| | Toughness : Breakage in coating | ++ | ++ | ++ | ++ | N/A |
| | Stain resistance : Δb | 9.3 | 12.1 | 7.6 | 2.4 | 42 |
| | | ++ | + | ++ | ++ | - |
| In the table, N/A means unmeasurable. | | | | | | |

As shown in Tables 2 to 5, the three-dimensional shaped articles coated with the energy ray-curable coating materials for three-dimensional shaped articles of the present invention in Examples 1 to 25 had surfaces with excellent smoothness, and the toughness was superior with the three-dimensional shaped articles retaining their inherent properties. The coating materials of the present invention are tough and not easily breakable, in addition to being flexible and highly stretchable. This enabled the coating materials of the present invention to conform to not only hard and fragile shaped articles such as (B)-1, but shaped articles that are flexible and undergo large deformation, such as (B)-2. The three-dimensional shaped article of Comparative Example 1 with no coating had surface irregularities from the lamination process. The three-dimensional shaped articles of Comparative Examples 2 to 4 coated with coating materials in which the polymerizable compounds were solely polyfunctional polymerizable compounds having a Mw/n of less than 120 had reduced tensile elongation as a result of penetration and curing of the coating materials in the shaped articles. In Comparative Examples 2 to 4, the coating material was unable to conform to flexible shaped articles such as (B)-2, and the coating (coating layer) of the coating material broke before breakage of the three-dimensional shaped articles. That is, the coating had low toughness, and impaired the inherent quality of the three-dimensional shaped articles. As shown in Table 3, the three-dimensional shaped articles coated with the energy ray-curable coating materials for three-dimensional shaped articles of the present invention in Examples 12 to 17 had excellent water resistance. As shown in Table 4, the three-dimensional shaped articles coated with the energy ray-curable coating materials for three-dimensional shaped articles of the present invention in Examples 18 to 21 had excellent antimicrobial properties. As shown in Table 5, the three-dimensional shaped articles coated with the energy ray-curable coating materials for three-dimensional shaped articles of the present invention in Examples 22 to 25 had excellent stain resistance.

## INDUSTRIAL APPLICABILITY

An energy ray-curable coating material for three-dimensional shaped articles of the present invention provides excellent toughness in the cured product, and is suited for coating flexible energy ray-curable three-dimensional shaped articles. A kit including the energy ray-curable coating material for three-dimensional shaped articles and an energy ray-curable composition for three-dimensional shaping provides excellent toughness in the cured product, and is particularly suited for an orthodontic mouthpiece, a mouthpiece for treating disorders, a mouthpiece for protecting teeth or temporomandibular joint, and a denture base material.

## Claims

1. An energy ray-curable coating material (A) for three-dimensional shaped articles, comprising a polymerizable compound and a polymerization initiator (c),

   the polymerizable compound comprising a monofunctional polymerizable compound (a), and/or a polyfunctional polymerizable compound (b) having two or more polymerizable groups per molecule,
   the polyfunctional polymerizable compound (b) having a Mw/n of 120 or more, where Mw is a molecular weight of the polyfunctional polymerizable compound (b), and n is the number of polymerizable groups per molecule.

2. The energy ray-curable coating material (A) for three-dimensional shaped articles according to claim 1, wherein the polymerization initiator (c) is a photopolymerization initiator (c1).

3. The energy ray-curable coating material (A) for three-dimensional shaped articles according to claim 1 or 2, wherein the polymerizable compound comprises the polyfunctional polymerizable compound (b), and the polyfunctional polymerizable compound (b) comprises a polyfunctional polymerizable compound having a Mw/n of 500 or more.

4. The energy ray-curable coating material (A) for three-dimensional shaped articles according to any one of claims 1 to 3, wherein the polymerizable compound comprises the monofunctional polymerizable compound (a), and the monofunctional polymerizable compound (a) is a radical polymerizable compound.

5. The energy ray-curable coating material (A) for three-dimensional shaped articles according to any one of claims 1 to 4, wherein the monofunctional polymerizable compound (a) comprises a monofunctional polymerizable compound (a1) having a polymer glass transition temperature of 60°C or more.

6. The energy ray-curable coating material (A) for three-dimensional shaped articles according to claim 5, wherein the monofunctional polymerizable compound (a1) is a (meth)acrylic compound having a side chain having at most two carbon atoms.

7. The energy ray-curable coating material (A) for three-dimensional shaped articles according to claim 5, wherein the monofunctional polymerizable compound (a1) is an aromatic (meth)acrylate compound.

8. The energy ray-curable coating material (A) for three-dimensional shaped articles according to any one of claims 1 to 7, wherein the monofunctional polymerizable compound (a) comprises a monofunctional polymerizable compound (a2) having a polymer glass transition temperature of less than 60°C.

9. The energy ray-curable coating material (A) for three-dimensional shaped articles according to any one of claims 1 to 7, wherein the polyfunctional polymerizable compound (b) comprises a polyfunctional polymerizable compound (b1) having a polymer glass transition temperature of 60°C or more.

10. The energy ray-curable coating material (A) for three-dimensional shaped articles according to claim 9, wherein the polyfunctional polymerizable compound (b1) comprises a polyfunctional polymerizable compound (b1-1) having no polymer backbone.

11. The energy ray-curable coating material (A) for three-dimensional shaped articles according to claim 9, wherein the polyfunctional polymerizable compound (b1) comprises a polyfunctional polymerizable compound (b1-2) having a polymer backbone.

12. The energy ray-curable coating material (A) for three-dimensional shaped articles according to any one of claims 1 to 11, wherein the monofunctional polymerizable compound (a) and/or the polyfunctional polymerizable compound (b) have at least one selected from an alicyclic hydrocarbon group, an aromatic hydrocarbon group, a chain alkyl group having 8 or more carbon atoms, a fluoro group, and a silyl group.

13. The energy ray-curable coating material (A) for three-dimensional shaped articles according to any one of claims 1 to 12, wherein the monofunctional polymerizable compound (a) and/or the polyfunctional polymerizable compound (b) have a zwitterionic group and/or a quaternary ammonium salt group.

14. The energy ray-curable coating material (A) for three-dimensional shaped articles according to any one of claims 1 to 13, which further comprises a solvent (d).

15. An energy ray-curable material kit for three-dimensional shaping, comprising an energy ray-curable coating material (A) for three-dimensional shaped articles of any one of claims 1 to 14, and an energy ray-curable composition (B) for three-dimensional shaping.

16. The energy ray-curable material kit for three-dimensional shaping according to claim 15, wherein the energy ray-curable composition (B) for three-dimensional shaping comprises a polymerizable compound and a polymerization initiator (c),

the polymerizable compound comprising a monofunctional polymerizable compound (a), and/or a polyfunctional polymerizable compound (b) having two or more polymerizable groups per molecule,
the polyfunctional polymerizable compound (b) having a Mw/n of 120 or more, where Mw is a molecular weight of the polyfunctional polymerizable compound (b), and n is the number of polymerizable groups per molecule.

17. The energy ray-curable material kit for three-dimensional shaping according to claim 15 or 16, wherein the difference between a transparency $\Delta L_A$ of a cured product of the energy ray-curable coating material (A) for three-dimensional shaped articles and a transparency $\Delta L_B$ of a cured product of the energy ray-curable composition (B) for three-dimensional shaping is 20 or less.

18. The energy ray-curable material kit for three-dimensional shaping according to any one of claims 15 to 17, wherein the difference between a tensile elastic modulus of a cured product of the energy ray-curable coating material (A) for three-dimensional shaped articles and a tensile elastic modulus of a cured product of the energy ray-curable composition (B) for three-dimensional shaping is 200 MPa or less.

19. A three-dimensional shaped article comprising a coating layer,

the three-dimensional shaped article being a cured product of an energy ray-curable composition (B) for three-dimensional shaping,

the coating layer being a cured product of an energy ray-curable coating material (A) for three-dimensional shaped articles of any one of claims 1 to 14.

**20.** The three-dimensional shaped article according to claim 19, wherein the coating layer formed of the energy ray-curable coating material (A) for three-dimensional shaped articles is 250 μm or less.

**21.** The three-dimensional shaped article according to claim 19 or 20, wherein a covalent bond is formed between the polymerizable compound in the energy ray-curable coating material (A) for three-dimensional shaped articles constituting the coating layer, and a polymerizable compound in the energy ray-curable composition (B) for three-dimensional shaping constituting the three-dimensional shaped article.

**22.** A method for producing a coated three-dimensional shaped article, comprising the steps of:

curing the energy ray-curable composition (B) for three-dimensional shaping included in the energy ray-curable material kit for three-dimensional shaping of any one of claims 15 to 18 to form a three-dimensional shaped article; and
coating the three-dimensional shaped article with the energy ray-curable coating material (A) for three-dimensional shaped articles included in the energy ray-curable material kit for three-dimensional shaping.

**23.** The method for producing a coated three-dimensional shaped article according to claim 22, which further comprises subjecting the three-dimensional shaped article coated with the energy ray-curable coating material (A) for three-dimensional shaped articles to photoirradiation and/or heat treatment to integrate the energy ray-curable coating material (A) for three-dimensional shaped articles and the three-dimensional shaped article of the energy ray-curable composition (B) for three-dimensional shaping.

**24.** The method for producing a coated three-dimensional shaped article according to claim 22 or 23, wherein the energy ray-curable composition (B) for three-dimensional shaping is cured by stereolithography.

**25.** The method for producing a coated three-dimensional shaped article according to any one of claims 22 to 24, wherein the three-dimensional shaped article is a dental material.

**26.** The method for producing a coated three-dimensional shaped article according to any one of claims 22 to 25, wherein the three-dimensional shaped article is an orthodontic mouthpiece, a mouthpiece for treating sleep disorders, a mouthpiece for treating temporomandibular joint disorders, or a mouthpiece for protecting teeth or temporomandibular joint.

**27.** The method for producing a coated three-dimensional shaped article according to claim 25, wherein the dental material is a denture base material.

## INTERNATIONAL SEARCH REPORT

International application No. PCT/JP2020/049041

A. CLASSIFICATION OF SUBJECT MATTER

C08F 220/10(2006.01)i; B33Y 10/00(2015.01)i; B33Y 80/00(2015.01)i; B29C 64/106(2017.01)i; B29C 64/259(2017.01)i; B29C 64/264(2017.01)i; A61K 6/30(2020.01)i; A61K 6/889(2020.01)i; B33Y 70/00(2020.01)i

FI: C08F220/10; B33Y70/00; B33Y80/00; B33Y10/00; B29C64/259; A61K6/889; A61K6/30; B29C64/264; B29C64/106

According to International Patent Classification (IPC) or to both national classification and IPC

B. FIELDS SEARCHED

Minimum documentation searched (classification system followed by classification symbols)

C08F220/10; B33Y10/00; B33Y80/00; B29C64/106; B29C64/259; B29C64/264; A61K6/30; A61K6/889; B33Y70/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2019/021451 A1 (NIKON CORPORATION) 31 January 2019 (2019-01-31) claims, examples | 1-2, 4-6, 9-12, 14-24<br>3, 7-8, 13, 25-27 |
| Y<br>A | JP 2016-210951 A (RICOH CO., LTD.) 15 December 2016 (2016-12-15) claims, paragraph [0040], examples | 1-6, 8, 10-12, 14, 19-20<br>7, 9, 13, 15-18, 21-27 |
| Y<br>A | JP 2016-088002 A (SEIKO EPSON CORP.) 23 May 2016 (2016-05-23) paragraph [0167] | 1-6, 8, 10-12, 14, 19-20<br>7, 9, 13, 15-18, 21-27 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>24 February 2021 (24.02.2021) | Date of mailing of the international search report<br>09 March 2021 (09.03.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/049041

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2016-078337 A (SEIKO EPSON CORP.) 16 May 2016 (2016-05-16) paragraph [0140] | 1-6, 8, 10-12, 14, 19-20<br>7, 9, 13, 15-18, 21-27 |
| A | JP 2000-318048 A (JSR CORPORATION, JAPAN FINE COATINGS CO., LTD.) 21 November 2000 (2000-11-21) entire text | 1-27 |

Form PCT/ISA/210 (continuation of second sheet) (January 2015)

**INTERNATIONAL SEARCH REPORT**
Information on patent family members

International application No.
PCT/JP2020/049041

| Patent Documents referred in the Report | Publication Date | Patent Family | Publication Date |
|---|---|---|---|
| WO 2019/021451 A1 | 31 Jan. 2019 | CN 110945039 A<br>pp. 2-3, 16-21 | |
| JP 2016-210951 A | 15 Dec. 2016 | US 2016/0326387 A1<br>claims, examples | |
| JP 2016-088002 A | 23 May 2016 | (Family: none) | |
| JP 2016-078337 A | 16 May 2016 | US 2016/0107385 A1<br>paragraph [0158] | |
| JP 2000-318048 A | 21 Nov. 2000 | WO 2000/067981 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000318048 A
- JP 2002305796 A
- WO 2006016649 A
- JP 2012072327 A
- WO 2018038056 A
- JP 2000159621 A